# EUROPÄISCHE PATENTSCHRIFT

(11) **EP 2 285 806 B1**
(45) Veröffentlichungstag und Bekanntmachung des Hinweises auf die Patenterteilung: **15.01.2014**
(21) Anmeldenummer: 09733860.2
(22) Anmeldetag: 21.04.2009
(51) Int. Cl.: C07D 471/06, C08K 5/3437

(54) **DREIFACH UND VIERFACH SUBSTITUIERTE PENTARYLENTETRACARBONSÄUREDIIMIDE**
THREEFOLD AND FOURFOLD SUBSTITUTED PENTARYLENE TETRACARBOXYLIC ACID DIIMIDES
DIMIIDES D ACIDE PENTARYLÈNETÉTRACARBOXYLIQUE TROIS ET QUATRE FOIS SUBSTITUÉS

(30) Priorität: 22.04.2008 EP 08154959
(43) Veröffentlichungstag der Anmeldung: 23.02.2011
(73) Patentinhaber: BASF SE, 67056 Ludwigshafen (DE)
(72) Erfinder: QU, Jianqiang, 67061 Ludwigshafen (DE); PSCHIRER, Neil, Gregory, 55116 Mainz (DE)
(86) Internationale Anmeldenummer: PCT/EP2009/054751
(87) Internationale Veröffentlichungsnummer: WO 2009/130223

(56) Entgegenhaltungen:
- WO-A-02/077081
- WO-A-2006/111511
- PSCHIRER N G ET AL: "Pentarylene- and Hexarylenebis(dicarboximide)s: Near- Infrared-Absorbing Polyaromatic Dyes" ANGEWANDTE CHEMIE. INTERNATIONAL EDITION, WILEY VCH VERLAG, WEINHEIM, Bd. 45, 1. Januar 2006 (2006-01-01), Seiten 1401-1404, XP002474592 ISSN: 1433-7851
- QUANTE H ET AL: "QUATERRYLENEBIS(DICARBOXIMIDES)" ANGEWANDTE CHEMIE. INTERNATIONAL EDITION, WILEY VCH VERLAG, WEINHEIM, Bd. 34, Nr. 12, 7. Juli 1995 (1995-07-07), Seiten 1323-1325, XP002002793 ISSN: 1433-7851

## Beschreibung

Die vorliegende Erfindung betrifft Pentarylentetracarbonsäurediimide, deren Herstellung sowie Vorstufen zur Herstellung dieser und deren Verwendung.

Rylentetracarbonsäurediimide sind bekanntermaßen aufgrund ihrer starken Absorption im Nahinfrarot (NIR-)-Bereich des elektromagnetischen Spektrums von besonderem anwendungstechnischen Interesse.

So wird zum Beispiel in WO-A 02/77081 der Einsatz von Quaterrylentetracarbonsäurediimiden als Infrarotabsorber für den Wärmeschutz in Glaslaminaten beschrieben.

Pentarylenderivate, die unsubstituiert sind oder einen geringen Substitutionsgrad aufweisen, sind von N. G. Pschirer et al., Angew. Chem. Int. Ed. 45 (2006), 1401-1404 beschrieben.

Ähnliche Pentarylenderivate sind auch in DE-A 10 2005 018241 beschrieben.

Trotz der bereits beschriebenen Pentarylenderivate sowie deren Verwendung im Zusammenhang mit deren Absorptionsvermögen im NIR besteht ein Bedarf an weiteren, insbesondere speziell substituierten Derivaten, die insbesondere einfach herzustellen sind.

Eine Aufgabe der vorliegenden Erfindung ist somit die Bereitstellung solcher Pentarylentetracarbonsäurediimide sowie Verfahren zu deren Herstellung, die insbesondere Vorzüge bei der Synthese und gute Eigenschaften aufgrund ihres Absorptionsvermögens aufweisen.

Diese Aufgabe wird gelöst durch ein Pentarylentetracarbonsäurediimid der Formel (I) oder (Ia) oder Mischungen davon. 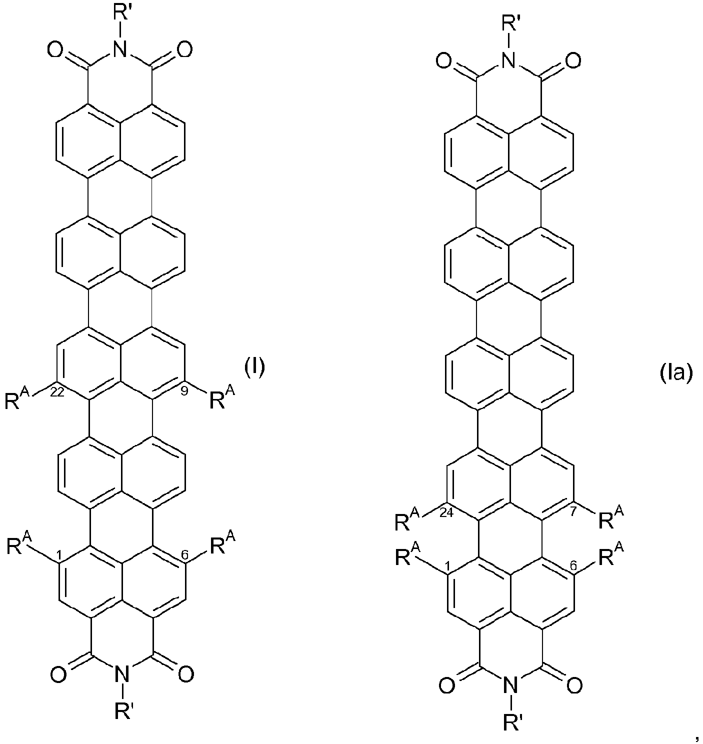 wobei
jedes R^{A} unabhängig voneinander gleich oder verschieden der folgende Rest ist:
   H;
   Aryloxy, Arylthio, Hetaryloxy oder Hetarylthio, an das jeweils weitere gesättigte oder ungesättigte 5- bis 7-gliedrige Ringe, deren Kohlenstoffgerüst durch eine oder mehrere Gruppierungen -O-, -S-, -NR¹-, -N=CR¹-,
      -CO-, -SO- und/oder -SO₂- unterbrochen sein kann, anneliert sein können, wobei das gesamte Ringsystem ein- oder mehrfach durch die Reste (i), (ii), (iii), (iv) und/oder (v) substituiert sein kann:
      (i) C₁-C₃₀-Alkyl, dessen Kohlenstoffkette durch eine oder mehrere Gruppierungen - O-, -S-, -NR¹-, -N=CR¹-, -C≡C-, -CR¹=CR¹-, -CO-, -SO- und/oder -SO₂- unterbrochen sein kann und das ein- oder mehrfach substituiert sein kann durch: C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR₂COR³, -CONR²R³, -SO₂NR²R³, -COOR², -SO₃R², -PR²R³ ,-POR²R³, Aryl und/oder gesättigtes oder ungesättigtes C₄-C₇-Cycloalkyl, dessen Kohlenstoffgerüst durch eine oder mehrere Gruppierungen -O-, -S-, -NR¹-, -N=CR¹-, -CR¹=CR¹-, -CO-, -SO-und/oder -SO₂- unterbrochen sein kann, wobei die Aryl- und Cycloalkylreste jeweils ein- oder mehrfach durch C₁-C₁₈-Alkyl und/oder die vorstehenden, als Substituenten für Alkyl genannten Reste substituiert sein können;
      (ii) C₃-C₈-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR¹-, - N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², - SO₃R², -PR²R³ und/oder -POR²R³;
      (iii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³, -POR²R³, Aryl und/oder Hetaryl, das jeweils durch C₁-C₁₈-Alkyl, C₁-C₁₂-Alkoxy, Hydroxy, Mercapto, Halogen, Cyano, Nitro, -NR²R³, -NR²COR³, - CONR²R³, -SO₂NR²R³, -COOR², -SO₃R², -PR²R³ und/oder -POR²R³ substituiert sein kann;
      (iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR¹-, -CO-, -SO- oder -SO₂ bedeutet;
      (v) C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², - PR²R³ und/oder -POR²R³;
   R¹ Wasserstoff oder C₁-C₁₈-Alkyl, wobei die Reste R¹ gleich oder verschieden sein können, wenn sie mehrfach auftreten, ist;
   R², R³ unabhängig voneinander Wasserstoff;
      C₁-C₁₈-Alkyl, dessen Kohlenstoffkette durch eine oder mehrere Gruppie-rungen -O-, -S-, -CO-, -SO- und/oder -SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder -COOR¹ substituiert sein kann;
   Aryl oder Hetaryl, an das jeweils weitere gesättigte oder ungesättigte 5- bis 7-gliedrige Ringe, deren Kohlenstoffgerüst durch eine oder mehrere Gruppierungen -O-, -S-, -CO-und/oder -SO₂- unterbrochen sein kann, anneliert sein können, wobei das gesamte Ringsystem ein- oder mehrfach durch C₁-C₁₂-Alkyl und/oder die vorstehenden, als Substituenten für Alkyl genannten Reste substituiert sein kann, sind;
jedes R' unabhängig voneinander Wasserstoff;
   C₁-C₃₀-Alkyl, dessen Kohlenstoffkette durch eine oder mehrere Gruppie-rungen -O-, -S-, -NR¹-, -N=CR¹-, -C≡C-, -CR¹=CR¹, -CO-, -SO- und/oder -SO₂- unterbrochen sein kann und das ein- oder mehrfach durch die als Substituenten für die Reste RA genannten Reste (ii), (iii), (iv) und/oder (v) substituiert sein kann;
   C₃-C₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder
      -SO₂- unterbrochen sein kann, anneliert sein können, wobei das gesamte Ringsystem durch die als Substituenten für die Reste R^{A} genannten Reste (i), (ii), (iii), (iv) und/oder (v) substituiert sein kann; oder
   Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder
      -SO₂- unterbrochen sein kann, anneliert sein können, wobei das gesamte Ringsystem substituiert sein kann durch die als Substituenten für die Reste R^{A} genannten Reste (i), (ii), (iii), (iv), (v), Aryl- und/oder Hetarylazo, das jeweils durch C₁-C₁₀-Alkyl, C₁-C₆-Alkoxy und/oder Cyano substituiert sein kann, ist;
mit der Maßgabe, dass mindestens drei R^{A} in Formel (I) bzw. (la) verschieden von H sind.

Diese Aufgabe wird weiterhin gelöst durch ein Verfahren zur Herstellung der erfindungsgemäßen Pentarylentetracarbonsäurediimide, die Schritte enthaltend
(a) Kupplung mindestens einer Terrylenverbindung der Formel (II) bzw. (IIa) 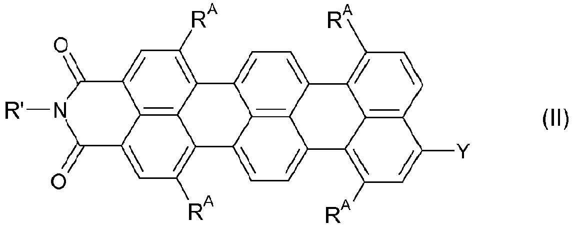 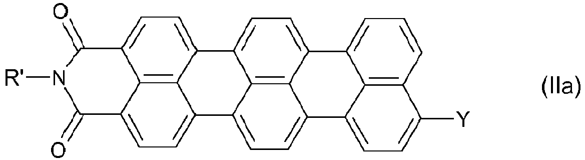 mit mindestens einer Verbindung der Formel (III) bzw. (IIIa) 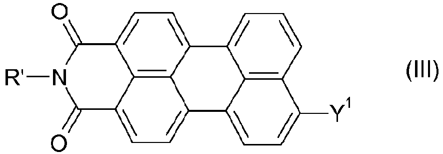 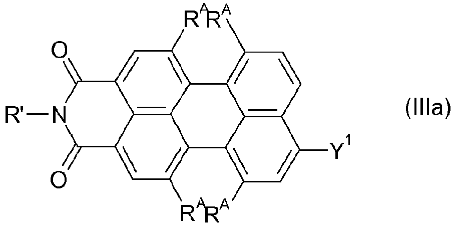 wobei
   Y, Y¹ beide Halogen sind oder ein Rest von Y, Y¹ Halogen und der andere B(OR")₂ ist;
   jedes R" unabhängig voneinander Wasserstoff, C₁-C₃₀-Alkyl, C₅-C₈-Cycloalkyl, Aryl oder Hetaryl oder miteinander verbunden unter Ausbildung eines die beiden Sauerstoffatome sowie das Boratom enthaltenden 5- bis 7-gliedrigen Rings, an den ungesättigte oder gesättigte Ringe anneliert sein können und der an den Kohlenstoffatomen durch bis zu 4 C₁-C₃₀-Alkyl-, C₅-C₈-Cycloalkyl-, Aryl- oder Hetarylgruppen substituiert sein kann, ist;
   jedes R' und R^{A} die Bedeutung wie in Anspruch 1 besitzt;
(b) Cyclodehydrierung des in Schritt (a) erhaltenen Umsetzungsproduktes zu einer Pentarylenverbindung der allgemeinen Formel (I) oder (la) oder Mischungen davon.

Es hat sich nämlich gezeigt, dass überraschenderweise spezielle tetrasubstituierte Pentarylentetracarbonsäurediimide erhalten hätten können, die in 1,6,9,22 bzw. in 1,6,7,24-Position einen Substituenten aufweisen sowie trisubstituierte Derivate, die in einer dieser Positionen unsubstituiert sind, wobei als Edukte jeweils zwei Ausgangsverbindungen ausgewählt werden, wobei die eine Ausgangsverbindung sämtliche Substituenten aufweist, so dass die andere in Bezug auf die Substitutenten R^{A} unsubstituiert ist. Diese Ausgangsstoffe stellen sehr preisgünstige Ausgangsstoffe dar und es konnte überraschenderweise eine Synthese erfolgen, ohne dass Löslichkeitsprobleme dadurch auftreten, dass ein Baustein unsubstituiert ist.

Bevorzugte Pentarylentetracarbonsäurediimide gemäß der vorliegenden Erfindung oder Mischungen davon sind solche, wobei alle vier Substituenten R^{A} in Formel (I) bzw. (la) verschieden von H sind.

Alternativ dazu ist bevorzugt, dass Pentarylentetracarbonsäurediimide der vorliegenden Erfindung oder Mischungen davon solche sind, bei denen R^{A} in 22-Position in Formel (I) oder R^{A} in 24-Position in Formel (la) H sind. Diese können zumindest teilweise dadurch erhalten werden, dass bei Schritt (b) des erfindungsgemäßen Verfahrens ausgehend von den tetrasubstituierten Verbindungen diese einen Substituenten R^{A} verlieren, so dass insbesondere Mischungen von drei- und vierfach substituierten Pentarylentetracarbonsäurediimide vorliegen können.

Bevorzugt sind Pentarylentetracarbonsäurediimide gemäß der vorliegenden Erfindung oder Mischungen davon gemäß Formel (I).

Die in Schritt (a) des erfindungsgemäßen Verfahrens zur Herstellung der efindungsgemäßen Pentarylentetracarbonsäurediimide erhaltenen Umsetzungsprodukte dienen als Ausgangsverbindungen in Schritt (b) des erfindungsgemäßen Verfahrens (Cyclodehydrierung).

Ein weiterer Gegenstand der vorliegenden Erfindung sind daher Pentarylentetracarbonsäurediimidvorstufen der Formel (Ib) oder (Ic) oder Mischungen davon 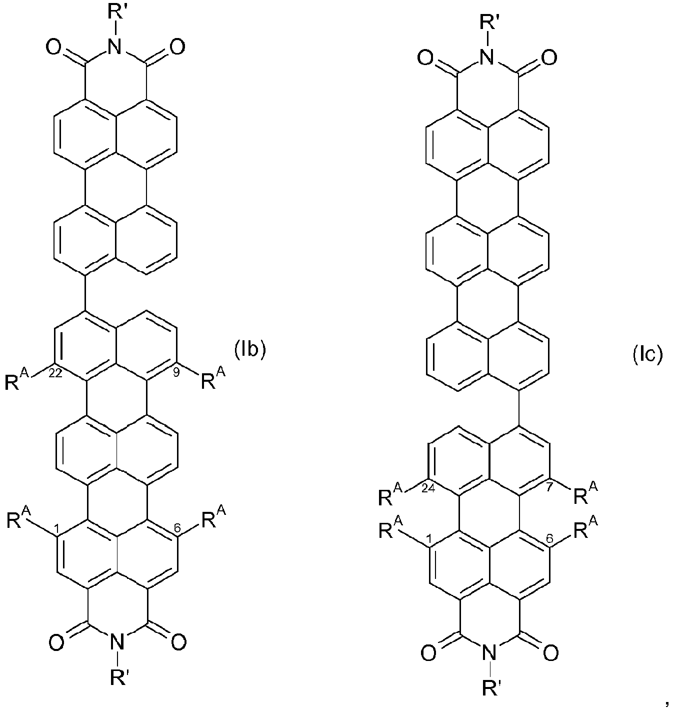 wobei jedes R' und R^{A} die Bedeutung wie in Anspruch 1 besitzt.

Die erfindungsgemäßen Pentarylenverbindungen können also mit Hilfe des erfindungsgemäßen Verfahrens durch Kupplung einer entsprechenden Terrylenverbindung mit der entsprechenden Perrylenverbindung erhalten werden. Die erfindungsgemäßen Pentarylenverbindungen weisen drei oder vier Substituenten R^{A} auf, die sich entsprechend an den in Formeln (I) und (la) angegebenen Positionen befinden.

Die Ausgangsverbindungen gemäß der Formeln (II), (III), (IIIa) sind aus dem Stand der Technik bekannt oder können anhand literaturbekannter Synthesen analoger Verbindungen hergestellt werden. Insbesondere Terrylenderivate, die als Ausgangsstoffe für das erfindungsgemäße Verfahren zur Herstellung von Pentarylentetracarbonsäurediimiden dienen können, sind in DE-A 10 2005 018241 beschrieben. Eine Herstellung der erfindungsgemäßen Imidverbindungen kann analog erfolgen.

Das erfindungsgemäße Verfahren zur Herstellung von Pentarylentetracarbonsäurediimiden enthält als ersten Schritt (a) die Kupplung mindestens einer Terrylenverbindung der Formel (II) oder (IIa) mit einer Verbindung der Formel (III) oder (IIIa), wobei die Verknüpfung der beiden Bausteine jeweils mit Hilfe der Gruppen Y und Y¹ erfolgt.

Dabei können Y und Y¹ Halogenide sein, durch die mit Hilfe einer katalytischen Kupplung die gewünschte Bindung der beiden aromatischen Bausteine ermöglicht wird. Ebenso ist es möglich, dass einer der Reste Y, Y¹ ein Halogenid und der andere eine Boronsäure oder eine ähnliche Verbindung der Formel B(OR")₂ sein kann. Eine Kupplung erfolgt dann über die sog. Suzuki-Reaktion. Vorzugsweise handelt es sich in beiden Fällen bei den Halogeniden um Bromid oder Chlorid.

Die Herstellung der erfindungsgemäßen Diimide mit Hilfe des erfindungsgemäßen Verfahrens erfolgt vorzugsweise in Gegenwart eines organischen Lösemittels, gewünschtenfalls im Gemisch mit Wasser, sowie eines Übergangsmetallkatalysators und einer Base, wobei wie oben ausgeführt wurde, einer der beiden Bausteine ein Boronäurederivat und der andere ein Halogenid darstellen kann. Ein solches Boronsäurederivat ist beispielsweise durch Umsetzung des entsprechenden halogenierten Aromaten mit Hilfe von Diboranen der allgemeinen Formel (IV) (R"O)₂B-B(OR")₂ in Gegenwart eines aprotischen organischen Lösungsmittels, eines Übergangsmetallkatalysators und einer Base erhältlich.

Geeignete Diborane der allgemeinen Formel (IV) sind insbesondere Bis(1,2- und 1,3-diolato)diborane, Tetraalkoxydiborane, Tetracycloalkoxydiborane und Tetra(het)aryloxydiborane sowie deren Mischformen. Als Beispiele für diese Verbindungen seien genannt: Bis(pinacolato)diboran, Bis(1,2-benzodiolato)-diboran, Bis(2,2-dimethyl-1,3-propandiolato)diboran, Bis(1,1,3,3-tetramethyl-1,3-pro-pandiolato)diboran, Bis(4,5-pinandiolato)diboran, Bis(tetramethoxy)diboran, Bis(tetra-cyclopentoxy)diboran, Bis(tetraphenoxy)diboran und Bis(4-pyridiyloxy)diboran.

Bevorzugt sind Diborane der allgemeinen Formel (IV), bei denen die beiden an einem Boratom befindlichen Reste R" unter Ausbildung eines die beiden Sauerstoffatome sowie das Boratom enthaltenden Fünf- oder Sechsrings miteinander verbunden sind. An die gebildeten Fünf- oder Sechsringe können aromatische oder gesättigte, auch bicyclische, Ringe, mit 5 bis 7 C-Atomen als Ringglieder anneliert sein. Alle Ringe bzw. Ringsysteme können durch bis zu 4 C₁-C₃₀-Alkyl-, C₅-C₈-Cycloalkyl-, Aryl- und/oder Hetarylreste substituiert sein, vorzugsweise sind sie durch bis zu 4 C₁-C₄-Alkylreste substituiert. Beispiele für diese bevorzugten Diborane sind die bereits oben genannten Bis(1,2- und 1,3-diolato)-diborane, wobei Bis(pinacolato)diboran besonders bevorzugt ist.

Das Molverhältnis Diboran der allgemeinen Formel (IV) zum halogenierten Aromaten liegt dabei im Allgemeinen bei 0,8 : 1 bis 3 : 1, insbesondere bei 1,5 : 1 bis 2 : 1.

Als Lösungsmittel sind grundsätzlich alle unter den Reaktionsbedingungen gegen Basen stabilen aprotischen Lösungsmittel mit einem Siedepunkt oberhalb der gewählten Reaktionstemperatur geeignet, in denen sich die Edukte bei Reaktionstemperatur vollständig und die verwendeten Katalysatoren und Basen zumindest partiell lösen, so dass weitgehend homogene Reaktionsbedingungen vorliegen. Es können sowohl unpolare aprotische als auch polare aprotische Lösungsmittel eingesetzt werden.

Beispiele für bevorzugte unpolar-aprotische Lösungsmittel sind bei > 100°C siedende Lösungsmittel aus den folgenden Gruppen: Aliphaten (insbesondere C₈-C₁₈-Alkane), unsubstituierte, alkylsubstituierte und kondensierte Cycloaliphaten (insbesondere unsubstituierte C₇-C₁₀-Cycloalkane, C₆-C₈-Cycloalkane, die durch ein bis drei C₁-C₆-Alkylgruppen substituiert sind, polycyclische gesättigte Kohlenwasserstoffe mit 10 bis 18 C-Atomen), alkyl- und cycloalkylsubstituierte Aromaten (insbesondere Benzol, das durch ein bis drei C₁-C₆-Alkylgruppen oder einen C₅-C₈-Cycloalkylrest substituiert ist) und kondensierte Aromaten, die alkylsubstituiert und/oder teilhydriert sein können (insbesondere Naphthalin, das durch ein bis vier C₁-C₆-Alkylgruppen substituiert ist) sowie Mischungen dieser Lösungsmittel.

Als Beispiele für besonders bevorzugte Lösungsmittel seien im einzelnen genannt: Octan, Isooctan, Nonan, Isononan, Decan, Isodecan, Undecan, Dodecan, Hexadecan und Octadecan; Cycloheptan, Cyclooctan, Methylcyclohexan, Dimethylcyclohexan, Trimethylcyclohexan, Ethylcyclohexan, Diethylcyclohexan, Propylcyclohexan, Isopro-pylcyclohexan, Dipropylcyclohexan, Butylcyclohexan, tert.-Butylcyclohexan, Methyl-cycloheptan und Methylcyclooctan; Toluol, o-, m- und p-Xylol, 1,3,5-Trimethylbenzol (Mesitylen), 1,2,4- und 1,2,3-Trimethylbenzol, Ethylbenzol, Propylbenzol, Isopropyl-benzol, Butylbenzol, Isobutylbenzol, tert.-Butylbenzol und Cyclohexylbenzol; Naphthalin, Decahydronaphthalin (Dekalin), 1- und 2-Methylnaphthalin und 1- und 2-Ethyl-naphthalin; Kombinationen aus den zuvor genannten Lösungsmitteln, wie sie aus den hochsiedenden, teil- oder durchhydrierten Fraktionen thermischer und katalytischer Crackprozesse bei der Rohöl- oder Naphthaverarbeitung gewonnen werden können, z.B. Gemische vom Exsol^{®} Typ und Alkylbenzolgemische vom Solvesso^{®} Typ.

Ganz besonders bevorzugte Lösungsmittel sind Xylol (alle Isomeren), Mesitylen und vor allem Toluol.

Beispiele für geeignete polar-aprotische Lösungsmittel sind N,N-disubstituierte aliphatische Carbonsäureamide (insbesondere N,N-Di-C₁-C₄-alkyl-C₁-C₄-carbonsäureamide), stickstoffhaltige Heterocyclen und aprotische Ether (insbesondere cyclische Ether, Diarylether und Di-C₁-C₆-alkylether von monomeren und oligomeren C₂-C₃-Alkylenglykolen, die bis zu 6 Alkylenoxideinheiten enthalten können, vor allem Diethylenglykoldi-C₁-C₄-alkylether).

Als Beispiele für besonders geeignete Lösungsmittel seien im einzelnen genannt: N,N-Dimethylformamid, N,N-Diethylformamid, N,N-Dimethylacetamid und N,N-Dimethyl-butyramid; N-Methyl-2-pyrrolidon, Chinolin, Isochinolin, Chinaldin, Pyrimidin, N-Methyl-piperidin und Pyridin; Tetrahydrofuran, Dioxan, Diphenylether, Diethylenglykoldimethyl-, -diethyl-, -dipropyl-, -diisopropyl-, -di-n-butyl-, -di-sec.-butyl- und -di-tert.-butylether, Diethylenglykolmethylethylether, Triethylenglykoldimethyl- und -diethylether und Triethylenglykolmethylethylether.

Die Lösungsmittelmenge beträgt in der Regel 10 bis 1000 ml, bevorzugt 20 bis 300 ml, pro g halogeniertem Aromaten.

Als Übergangsmetallkatalysator eignen sich insbesondere Palladiumkomplexe, die wiederum in der Regel in Mengen von 1 bis 20 mol%, vor allem 2 bis 10 mol-%, bezogen auf den halogenierten Aromaten eingesetzt werden.

Beispiele für solche Katalysatoren sind Tetrakis(triphenylphosphin)palladium(0), Tetrakis(tris-o-tolylphosphin)palladium(0), [1,2-Bis(diphenylphosphino)ethan]palladium(II)-chlorid, [1,1'-Bis(diphenylphosphino)-ferrocen]palladium(II)chlorid, Bis(triethyl-phosphin)palladium(II)chlorid, Bis(tricyclo-hexylphosphin)palladium(II)acetat, (2,2'-Bipyridyl)palladium(II)chlorid, Bis(triphenyl-phosphin)palladium(II)chlorid, Tris(dibenzylidenaceton)dipalladium(0), 1,5-Cycloocta-dienpalladium(II)chlorid, Bis(acetonitril)palladium(II)chlorid und Bis(benzonitril)palla-dium(II)chlorid, Palladium(II)acetat, wobei [1,1'Bis(diphenylphosphino)ferrocen]palladium(II)chlorid, Tetrakis(triphenylphosphin)palladium(0) und Palladium(II)acetat bevorzugt sind.

In der Regel empfiehlt sich die gleichzeitige Anwesenheit freier Ligandmoleküle, z.B. von Tri(tert.-butyl)phosphin, Tri(i-butyl)phosphin, Triphenylphosphin und Tris(o-tolyl)phosphin und 2-Dicyclohexylphosphino-2,6-dimethoxybiphenyl. Übliche Mengen liegen bei 80 bis 500 mol-%, bevorzugt 100 bis 300 mol-%, bezogen auf den Übergangsmetallkatalysator.

Als Base kommen vorzugsweise die Alkalimetallsalze, insbesondere die Natrium- und vor allem die Kaliumsalze, schwacher organischer und anorganischer Säuren, wie Natriumacetat, Kaliumacetat, Natriumcarbonat, Natriumhydrogencarbonat, Kaliumcarbonat und Kaliumhydrogencarbonat, Phosphate, Fluoride wie Kaliumfluorid, zum Einsatz. Bevorzugte Basen sind die Acetate, vor allem Kaliumacetat.

Im Allgemeinen werden 1 bis 5 mol, bevorzugt 2 bis 4 mol, Base pro mol halogeniertem Aromaten verwendet.

Die Reaktionstemperatur liegt üblicherweise bei 20 bis 180°C, vor allem bei 60 bis 120°C.

Die Reaktionszeit beträgt in der Regel 0,5 bis 30 h, insbesondere 1 bis 20 h.

Vefahrenstechnisch geht man bei der Herstellung der Boronsäurederivate zweckmäßigerweise wie folgt vor:
Man legt den halogenierten Aromaten und Lösungsmittel vor, gibt das Diboran der allgemeinen Formel (IV), den Übergangsmetallkatalysator und die Base nacheinander zu und erhitzt die Mischung 0,5 bis 30 h unter Schutzgas auf die gewünschte Reaktionstemperatur. Nach Abkühlen auf Raumtemperatur filtriert man die festen Bestandteile aus dem Reaktionsgemisch ab und destilliert das Lösungsmittel unter vermindertem Druck ab.

Die Suzuki-Reaktion des so hergestellten Boronsäurederivates mit dem entsprechenden halogenierten Aromaten kann grundsätzlich zur Herstellung des Produktes in Schritt (a) des erfindungsgemäßen Verfahrens unter analogen Bedingungen verwendet werden, wobei an Stelle des Diborans das entsprechende Boronsäurederivat mit dem entsprechenden halogenierten Aromaten umgesetzt wird.

Vorzugsweise erfolgt jedoch die Umsetzung des Boronsäurederivates mit dem halogenierten Aromaten in Gegenwart eines organischen Lösungsmittels, gewünschtenfalls im Gemisch mit Wasser, sowie eines Übergangsmetallkatalysators und einer Base durchgeführt, wobei das Molverhältnis von Boronsäurederivat zu halogeniertem Aromaten dabei in der Regel 0,8 : 1 bis 3 : 1, vorzugsweise 0,9 : 1 bis 2 : 1 beträgt.

Als Lösungsmittel eignen sich alle Lösungsmittel, in denen sich die Edukte bei Reaktionstemperatur vollständig und die verwendeten Katalysatoren und Basen zumindest partiell lösen, so dass weitgehend homogene Reaktionsbedingungen vorliegen. Beispielsweise geeignet sind Octan, Isooctan, Nonan, Isononan, Decan, Isodecan, Undecan, Dodecan, Hexadecan und Octadecan; Cycloheptan, Cyclooctan, Methylcyclohexan, Dimethylcyclohexan, Trimethylcyclohexan, Ethylcyclohexan, Diethylcyclohexan, Propylcyclohexan, Isopropylcyclohexan, Dipropylcyclohexan, Butylcyclohexan, tert.-Butylcyclohexan, Methyl-cycloheptan und Methylcyclooctan; Toluol, o-, m- und p-Xylol, 1,3,5-Trimethylbenzol (Mesitylen), 1,2,4-und 1,2,3-Trimethylbenzol, Ethylbenzol, Propylbenzol, Isopropylbenzol, Butylbenzol, Isobutylbenzol, tert.-Butylbenzol und Cyclohexylbenzol; Naphthalin, Decahydronaphthalin (Dekalin), 1- und 2-Methylnaphthalin und 1- und 2-Ethyl-naphthalin; Kombinationen aus den zuvor genannten Lösungsmitteln, wie sie aus den hochsiedenden, teil- oder durchhydrierten Fraktionen thermischer und katalytischer Crackprozesse bei der Rohöl- oder Naphthaverarbeitung gewonnen werden können, z.B. Gemische vom Exsol^{®} Typ und Alkylbenzolgemische vom Solvesso^{®} Typ.

Ganz besonders bevorzugte Lösungsmittel sind Xylol (alle Isomeren), Mesitylen und vor allem Toluol.

Die Lösungsmittelmenge liegt üblicherweise bei 10 bis 1000 ml, vorzugsweise bei 20 bis 100 ml, je g Boronsäurederivat.

Vorzugsweise setzt man Wasser als zusätzliches Lösungsmittel ein. In diesem Fall werden in der Regel 10 bis 1000 ml, insbesondere 250 bis 500 ml, Wasser je I organisches Lösungsmittel verwendet.

Als Übergangsmetallkatalysatoren werden ebenfalls vorzugsweise Palladiumkomplexe eingesetzt. Die Einsatzmenge Katalysator beträgt üblicherweise 1 bis 20 mol-%, insbesondere 1,5 bis 5 mol-%, bezogen auf das Boronsäurederivat.

In der Regel empfiehlt sich die gleichzeitige Anwesenheit freier Ligandmoleküle, z.B. von Tri(tert.-butyl)phosphin, Tri(i-butyl)phosphin, Triphenylphosphin und Tris(o-tolyl)phosphin und 2-Dicyclohexylphosphino-2,6-dimethoxybiphenyl. Übliche Mengen liegen bei 80 bis 500 mol-%, bevorzugt 100 bis 300 mol-%, bezogen auf den Übergangsmetallkatalysator.

Als Base sind Alkalimetallsalze schwacher Säuren bevorzugt, wobei die Carbonate, wie Natriumcarbonat und vor allem Kaliumcarbonat besonders bevorzugt sind. Auch Phosphate, wie Natrium- oder Kaliumphosphat, sind hierbei ebenfalls bevorzugt. In der Regel liegt die Basenmenge bei 0,1 bis 10 mol, insbesondere bei 0,2 bis 5 mol, je mol Boronsäurederivat.

Die Reaktionstemperatur beträgt im Allgemeinen 20 bis 180°C, bevorzugt 60 bis 120°C. Wird in Schritt b) Wasser eingesetzt, so empfiehlt es sich, die Umsetzung nicht bei Temperaturen über 100°C vorzunehmen, da ansonsten unter Druck gearbeitet werden müsste.

Die Reaktion ist üblicherweise in 0,5 bis 48 h, insbesondere in 5 bis 20 h, beendet.

Verfahrenstechnisch geht man zweckmäßigerweise wie folgt vor:
Man legt das Boronsäurederivat und den halogenierten Aromaten sowie Lösungsmittel vor, gibt Übergangsmetallkatalysator und die vorzugsweise in Wasser oder einem Wasser/Alkohol-Gemisch gelöste Base zu und erhitzt die Mischung 0,5 bis 48 h unter Schutzgas auf die gewünschte Reaktionstemperatur. Nach Abkühlen auf Raumtemperatur trennt man die organische Phase aus dem Reaktionsgemisch ab und destilliert das Lösungsmittel unter vermindertem Druck ab.

Die Reinheit des so hergestellten Produktes aus Schritt (a) des erfindungsgemäßen Verfahrens zur Herstellung der Pentarylentetracarbonsäurediimide reicht im allgemeinen für die weitere Umsetzung in Schritt (b) aus. Gegebenenfalls kann das Rohprodukt durch Waschen mit Wasser und gewünschtenfalls einem geeigneten organischen Lösungsmittel, insbesondere einem chlorierten aliphatischen oder aromatischen Kohlenwasserstoff, oder durch Säulenchromatographie an Kieselgel mit einem Gemisch von Methylenchlorid und Hexan oder Pentan oder mit Toluol als Eluens weiter aufgereinigt werden.

Die Ausbeute in Schritt a) des erfindungsgemäßen Verfahrens liegt üblicherweise bei 20 bis 95%.

Neben der oben beschriebenen Suzuki-Reaktion, die ein entsprechendes Boronsäurederivat erfordert, kann auch eine direkte Kupplung, insbesondere bei Homokupplungen, von Halogeniden erfolgen.

Hierbei kann die Umsetzung der entsprechend halogenierten Aromaten der Formel (II) bzw. (IIa) und (III) bzw. (IIIa) in Gegenwart eines Diborans der allgemeinen Formel (IV) erfolgen. Dabei läuft schließlich ebenfalls eine Suzuki-Reaktion ab, wobei jedoch das entsprechende Boronsäurederivat nur in situ erzeugt wird.

Die Kupplung kann dabei beispielsweise in Gegenwart von 30 bis 70 mol-%, bezogen auf das den halogenierten Aromaten, eines Diborans der allgemeinen Formel (IV), eines Übergangsmetallkatalysators, einer Base und eines aprotischen Lösungsmittels gemäß einer Suzuki-Kupplungsreaktion erfolgen, wobei das in situ gebildete Boronsäurederivat nicht zwischenisoliert, sondern direkt mit dem verbleibenden halogenierten Aromaten umgesetzt wird.

Bei dieser Verfahrensvariante wird analog wie oben vorgegangen, wobei jedoch beispielsweise nur 30 bis 70 mol-% Diboran der allgemeinen Formel (IV), bezogen auf den halogenierten Aromaten eingesetzt werden.

In der Regel werden 1 bis 20 mol-%, bevorzugt 5 bis 10 mol-%, Übergangsmetallkatalysator und 1 bis 5 mol, vorzugsweise 2 bis 3 mol, Base je mol halogeniertem Aromaten verwendet. Das aprotische organische Lösungsmittel kommt üblicherweise in Mengen von 10 bis 100 ml, insbesondere 20 bis 50 ml, je g halogeniertem Aromaten zum Einsatz.

Die Reaktionstemperatur liegt im Allgemeinen bei 20 bis 100°C, vorzugsweise bei 60 bis 80°C, und die Reaktionsdauer bei 12 bis 72 h, bevorzugt 24 bis 48 h.

Verfahrenstechnisch geht man zweckmäßigerweise wie folgt vor:
Man legt den halogenierten Aromaten und Lösungsmittel vor, gibt das Diboran der allgemeinen Formel (IV), den Übergangsmetallkatalysator und die Base nacheinander zu und erhitzt die Mischung 12 bis 72 h auf die gewünschte Reaktionstemperatur. Nach Abkühlen auf Raumtemperatur trennt man die organische Phase aus dem Reaktionsgemisch ab und destilliert das Lösungsmittel unter vermindertem Druck ab.

Auch hier reicht die Reinheit des erhaltenen Produktes in der Regel für die nachfolgende Cyclodehydrierung in Schritt (b) des erfindungsgemäßen Verfahrens aus. Eine weitere Aufreinigung ist beispielsweise durch Säulenchromatographie möglich.

Die Ausbeute liegt üblicherweise bei 80 bis 95%.

Darüber hinaus besteht die Möglichkeit eine direkte Kupplung der halogenierten Aromaten (Halogenverbindung) durchzuführen, ohne dass ein Diboran eingesetzt wird.

Diese Kupplung kann dabei beispielsweise in Gegenwart eines organischen Übergangmetallkomplexes als Katalysator, freier Ligandmoleküle und eines aprotischen Lösungsmittels im Sinne einer Homo-Kupplung erfolgen.

Geeignete inerte Verdünnungsmittel sind zum Beispiel aliphatische Carbonsäureamide, wie N,N-Dimethylformamid und N, N-Dimethylacedamid, aliphatische und cycloaliphatische Ether, wie 1,2-Dimethoxyethan, und Aromaten, wie Benzol, Toluol und Xylol, wobei N, N-Dimethylformamid und N, N-Dimethylacedamid bevorzugt sind.

Die Verdünnungsmittelmenge beträgt in der Regel 20 bis 100 g, vorzugsweise 25 bis 45 g je Gramm Halogenverbindung.

Bei den als Katalysator dienenden organischen Übergangsmetallkomplexen kommen neben den bekannten Palladiumkomplexen, wie Tetrakis(triphenylphospin)palladium(0) insbesondere Nickelkomplexe in Betracht, zum Beispiel Bis(triphenylphosphin)nickel(II)chlorid, Tetrakis(triphenylphosphin)nickel(0), [1,2-Bis(diphenylphosphino)etan]nickel(II)chlorid und bevorzugt Bis(1,5-cyclooctadien)nickel(0). Man kann die Katalysatoren auch durch die Zugabe von Übergangsmetallsalzen oder - Verbindungen, freien Liganden wie Cyclooctadien, Bipyridyl, Triphenylphosphin, Trifluorphosphin, -η, ö- und π-gebundenen Olefine, Cycloolefine, Aromaten und Antiaromaten, Carbonylen, Wasserstoff und Halogen sowie auch deren Mischungen und erforderlichenfalls Oxidations- und Reduktionsmitteln erzeugen.

Im Allgemeinen werden 40 bis 150 mol-%, vorzugsweise 50 bis 100 mol-%, organischer Übergangsmetallkomplex bezogen auf die eingesetzte Halogenverbindung eingesetzt.

In der Regel empfiehlt sich stets die gleichzeitige Anwesenheit von freien Ligandmolekülen, wie insbesondere Mischungen von Cyclooctadien und Bipyridyl im molaren Verhältnis von 1 : 1 bis 8 : 1. Hierbei sind Mengen von üblicherweise 80 bis 900 mol-%, bevorzugt 80 bis 200 mol-%, bevorzugt bezogen auf die Halogenverbindung geeignet.

Die Kupplungstemperatur beträgt im Allgemeinen 40 bis 80°C, vorzugsweise 60 bis 70°C.

Die Reaktionszeit liegt in der Regel bei 24 bis 48 h, insbesondere bei 36 bis 48 h.

Verfahrenstechnisch geht man bei dieser direkten Kupplung zweckmäßigerweise so vor, dass man die Halogenverbindung, den metallorganischen Katalysator sowie freie Ligandmoleküle im inerten Verdünnungsmittel vorlegt und, ggf. unter Schutzgas 24 bis 48 h auf die gewünschte Reaktionstemperatur erhitzt. Nach Abkühlen trägt man das Reaktionsgemisch in Wasser, das ggf. Methanol enthalten kann, ein, gibt verdünnte anorganische Säure, z. B. verdünnte Salzsäure, zu und filtriert den gebildeten Niederschlag ab, wäscht mit Wasser und trocknet im Vakuum.

Die Reinheit des so hergestellten erfindungsgemäßen Produktes reicht im Allgemeinen für die nachfolgende Cyclodehydrierung in Schritt (b) des erfindungsgemäßen Verfahrens aus. Gegebenenfalls kann das Produkt noch zusätzlich durch eine Säulenchromatographie an Kieselgel mit einem Gemisch von Methylenchlorid und Hexan oder Pentan als Elluenz weiter aufgereinigt werden.

Die Ausbeute liegt im Allgemeinen bei 20 bis 60%.

In Schritt (b) des erfindungsgemäßen Verfahrens findet die Cylclodehydrierung des in Schritt (a) erhaltenen Umsetzungsprodukts statt. Die Cyclodehydrierung kann in einem Hydroxy- und Aminofunktionen aufweisenden und eine im Wesentlichen ungelöste Base enthaltenden organischen Reaktionsmedium oder in Gegenwart eines basenstabilen, hochsiedenden, organischen Lösemittels sowie einer alkali- oder erdalkalimetallhaltigen Base und einer stickstoffhaltigen Hilfsbase vorgenommen werden.

Bevorzugt ist die erstgenannte Verfahrensvariante. Hierbei sind als organisches Reaktionsmedium vor allem Aminoalkohole geeignet, die 2 bis 20, vorzugsweise 2 bis 10 Kohlenstoffatome aufweisen. Die Kohlenstoffkette dieser Alkohole kann durch Sauerstoffatome in Etherfunktion unterbrochen sein. Beispiele für besonders geeignete Lösungsmittel sind Ethanolamin, Triethanolamin und Diethanolamin, wobei Ethanolamin bevorzugt ist. Es ist auch möglich, Mischungen von Alkoholen und Aminen zu verwenden, die jeweils einen Siedepunkt von mindestens 70°C haben und bei der Reaktionstemperatur flüssig sind. Auch entsprechende Glykole, Mono- und Dialkylglykolether können alternativ oder in einem Gemisch zu den oben genannten Medien eingesetzt werden. Hierbei können Mono-, Di-, Tri-, Oligo- oder Polyalkylenglykole oder - glykolether verwendet werden. Beispiele sind Ethylenglykol, Ethylenglykolmonoethylether, Ethylenglykoldiethylether, Diethylenglykolmonoethylether oder Diethylenglykoldiethylether.

Üblicherweise werden 1,5 bis 150 ml, bevorzugt 5 bis 50 ml, Reaktionsmedium je Gramm Ausgangsverbindung eingesetzt.

Als im Reaktionsmedium wesentlich unlösliche Base eignen sich die Alkalimetallsalze, insbesondere die Natriumsalze und v.a. die Kaliumsalze, schwacher organischer und bevorzugt schwacher anorganischer Säuren, wie Formiate, Acetate, Propionate, Hydrogencarbonate und besonders bevorzugt Karbonate, insbesondere Natriumkarbonat und v.a. Kaliumkarbonat.

In der Regel beträgt die Basenmenge 1 bis 10 mol, bevorzugt 2 bis 5 mol, je Mol Ausgangsverbindung.

Die Reaktionstemperatur liegt im Allgemeinen bei 40 bis 200°C, insbesondere bei 80 bis 160°C.

Die Reaktionszeit beträgt üblicherweise 0,5 bis 64 h, vorzugsweise 1 bis 12 h.

Verfahrenstechnisch geht man zweckmäßigerweise so vor, dass man eine Mischung Ausgangsverbindung, Lösungsmittel und Base 0,5 bis 24 h unter Schutzgas bei der gewünschten Reaktionstemperatur rührt und das gebildete erfindungsgemäße Produkt der Formel (I) oder (la) nach Abkühlen auf Raumtemperatur durch Zugabe eines Alkohols, wie Ethanol, oder von Wasser aus dem Reaktionsgemisch ausfällt, abfiltriert und mit Wasser wäscht.

Die Reinigung der erfindungsgemäßen Rylenverbindung kann dadurch erfolgen, dass Katalysatorrückstände durch eine schnelle Filtration über Kieselgel unterwaschen mit einem halogenierten aliphatischen Kohlenwasserstoff, wie Methylenchlorid, entfernt werden. Rückstände nicht umgesetzter Edukte können durch Säulenchromatographie an Kieselgel mit Methylenchlorid als Eluenz oder durch wiederholtes Waschen mit Hexan oder Pentan entfernt werden.

Die Ausbeute liegt im Allgemeinen bei 50 bis 100%.

Als Produkt nach dem erfindungsgemäßen Verfahren zur Herstellung von Pentarylentetracarbonsäuredimiden werden Diimide der allgemeinen Formel (I) oder (la) 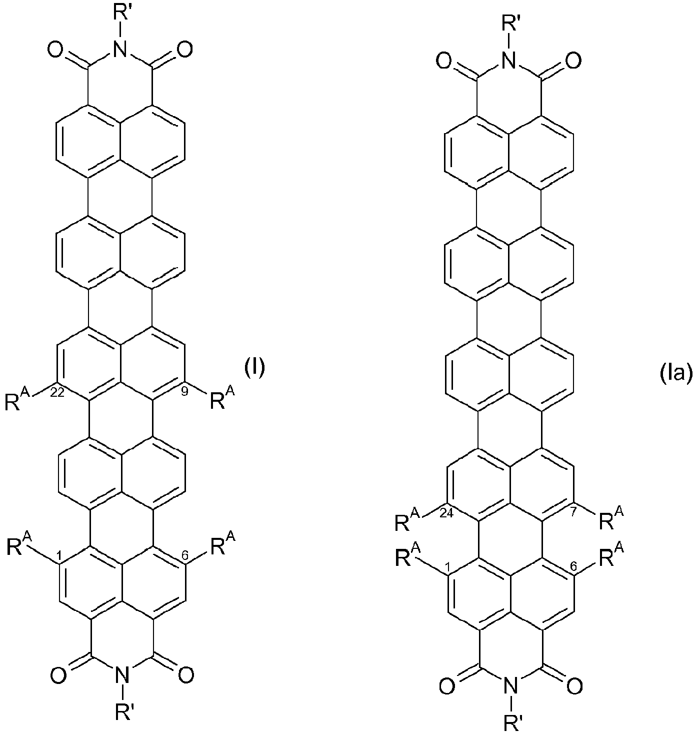 oder deren Mischungen erhalten. Hierbei haben R^{A,}, R' die oben angegebene Bedeutung.

Als Beispiele für die in den Formeln genannten Reste R^{A}, R', R", R¹ bis R³ sowie deren Substituenten seien im Einzelnen genannt:
Beispiele für Alkyle sind Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen); Die als Indizes angegebenen Zahlen nach dem Symbol "C" beziehen sich auf die Mindest- und Höchstanzahl der C-Atome der Alkyle.
Beispiele für durch Sauerstoff unterbrochene Alkyle sind 2-Methoxyethyl, 2-Ethoxyethyl, 2-Propoxyethyl, 2-Isopropoxyethyl, 2-Butoxyethyl, 2- und 3-Methoxypropyl, 2- und 3-Ethoxypropyl, 2- und 3-Propoxypropyl, 2- und 3-Butoxypropyl, 2- und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
Beispiele für durch Schwefel unterbrochene Alkyle sind 2-Methylthioethyl, 2-Ethylthioethyl, 2-Propylthioethyl, 2-Isopropylthioethyl, 2-Butylthio-ethyl, 2- und 3-Methylthiopropyl, 2- und 3-Ethylthiopropyl, 2- und 3-Propylthiopropyl, 2- und 3-Butylthiopropyl, 2- und 4-Methylthiobutyl, 2- und 4-Ethylthiobutyl, 2- und 4-Propylthiobutyl, 3,6-Dithiaheptyl, 3,6-Dithiaoctyl, 4,8-Dithianonyl, 3,7-Dithiaoctyl, 3,7-Di-thianonyl, 2- und 4-Butylthiobutyl, 4,8-Dithiadecyl, 3,6,9-Trithiadecyl, 3,6,9-Trithia-undecyl, 3,6,9-Trithiadodecyl, 3,6,9,12-Tetrathiatridecyl und 3,6,9,12-Tetrathiatetradecyl;
Beispiele für durch Aminogruppen unterbrochene Alkyle sind 2-Monomethyl- und 2-Monoethylaminoethyl, 2-Dimethylaminoethyl, 2- und 3- Dimethylaminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Diazaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
Weitere Beispiele für Alkylgruppen, die unterbrochen sind und/oder Substituenten aufweisen, sind
(1-Ethylethyliden)aminoethylen, (1-Ethylethyliden)aminopropylen, (1-Ethylethyliden)-aminobutylen, (1-Ethylethyliden)aminodecylen und (1-Ethylethyliden)aminododecylen;
Propan-2-on-1-yl, Butan-3-on-1-yl, Butan-3-on-2-yl und 2-Ethylpentan-3-on-1-yl;
2-Methylsulfoxidoethyl, 2-Ethylsulfoxidoethyl, 2-Propylsulfoxidoethyl, 2-Isopropylsulfoxidoethyl, 2-Butylsulfoxidoethyl, 2- und 3-Methylsulfoxidopropyl, 2- und 3-Ethylsulfoxidopropyl, 2- und 3-Propylsulfoxidopropyl, 2- und 3-Butylsulfoxidopropyl, 2- und 4-Methylsulfoxidobutyl, 2- und 4-Ethylsuffoxidobutyl, 2- und 4-Propylsulfoxidobutyl und 4-Butylsulfoxidobutyl;
2-Methylsulfonylethyl, 2-Ethylsulfonylethyl, 2-Propylsulfonylethyl, 2-Isopropylsulfonylethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylproypl, 2- und 4-Methylsulfonylbutyl, 2- und 4-Ethylsulfonylbutyl, 2- und 4-Propylsulfonylbutyl und 4-Butylsulfonylbutyl;
Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxytetradecyl;
Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Sulfododecyl und 14-Sulfotetradecyl;
2-Hydroxyethyl, 2- und 3-Hydroxypropyl, 1-Hydroxyprop-2-yl, 3- und 4-Hydroxybutyl, 1-Hydroxybut-2-yl und 8-Hydroxy-4-oxaoctyl;
2-Cyanoethyl, 3-Cyanopropyl, 3- und 4-cyanobutyl, 2-Methyl-3-ethyl-3-cyanopropyl, 7Cyano-7-ethylheptyl und 4-Methyl-7-methyl-7-cyanoheptyl;
2-Chlorethyl, 2- und 3-Chlorpropyl, 2-, 3- und 4-Chlorbutyl, 2-Bromethyl, 2- und 3-Brompropyl und 2-, 3- und 4-Brombutyl;
2-Nitroethyl, 2- und 3-Nitropropyl und 2-, 3- und 4-Nitrobutyl;
Beispiele für Alkyloxy sind Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy;
Beispiele für Alkylthio sind Methylthio, Ethylthio, Propylthio, Isopropylthio, Butylthio, Isobutylthio, sec.-Butylthio, tert.-Butylthio, Pentylthio, Isopentylthio, Neopentylthio, tert.-Pentylthio und Hexylthio;
Beispiele für Reste mit Dreifachbindung sind Ethinyl, 1- und 2-Propinyl, 1-, 2- und 3-Butinyl, 1-, 2-, 3- und 4-Pentinyl, 1-, 2-, 3-, 4- und 5-Hexinyl, 1-, 2-, 3-, 4-, 5-, 6-, 7-, 8- und 9-Decinyl, 1-, 2-, 3-, 4-, 5-, 6-, 7-, 8-, 9-, 10- und 11-Dodecinyl und 1-, 2-, 3-, 4-, 5-, 6-, 7-, 8-, 9-, 10-, 11-, 12-, 13-, 14-, 15-, 16- und 17-Octadecinyl;
Beispiele für Reste mit Doppelbindung sind Ethenyl, 1- und 2-Propenyl, 1-, 2- und 3-Butenyl, 1-, 2-, 3- und 4-Pentenyl, 1-, 2-, 3-, 4- und 5-Hexenyl, 1-, 2-, 3-, 4-, 5-, 6-, 7-, 8- und 9-Decenyl, 1-, 2-, 3-, 4-, 5-, 6-, 7-, 8-, 9-, 10- und 11-Dodecenyl und 1-, 2-, 3-, 4-, 5-, 6-, 7-, 8-, 9-, 10-, 11-, 12-, 13-, 14-, 15-, 16- und 17-Octadecenyl;
Beispiele für weitere Reste sind
Methylamino, Ethylamino, Propylamino, Isopryplamino, Butylamino, Isobutylamino, Pentylamino, Hexylamino, Dimethylamino, Methylethylamino, Diethylamino, Dipropylamino, Diisopropylamino, Dibutylamino, Diisobutylamino, Dipentylamino, Dihexylamino, Dicyclopentylamino, Dicyclohexylamino, Dicycloheptylamino, Diphenylamino und Dibenzylamino;
Formylamino, Acetylamino, Propionylamino und Benzoylamino;
Carbamoyl, Methylaminocarbonyl, Ethylaminocarbonyl, Propylaminocarbonyl, Butylaminocarbonyl, Pentylaminocarbonyl, Hexylaminocarbonyl, Heptylaminocarbonyl, Octylaminocarbonyl, Nonylaminocarbonyl, Decylaminocarbonyl und Phenylaminocarbonyl;
Aminosulfonyl, N,N-Dimethylaminosulfonyl, N,N-Diethylaminosulfonyl, N-Methyl-Nethylaminosulfonyl, N-Methyl-N-dodecylaminosulfonyl, N-Dodecylaminosulfonyl, (N,NDimethylamino)ethylaminosulfonyl, N,N-(Propoxyethyl)dodecylaminosulfonyl, N,NDiphenylaminosulfonyl, N,N-(4-tert.-Butylphenyl)octadecylaminosulfonyl und N,N-Bis(4-Chlorphenyl)aminosulfonyl;
Methoxycarbonyl, Ethoxycarbonyl, Propoxycarbonyl, Isopropoxycarbonyl, Hexoxycarbonyl, Dodecyloxycarbonyl, Octadecyloxycarbonyl, Phenoxycarbonyl, (4-tert.-Butyl-phenoxy)carbonyl und (4-Chlorphenoxy)carbonyl;
Methoxysulfonyl, Ethoxysulfonyl, Propoxysulfonyl, Isopropoxysulfonyl, Butoxysulfonyl, Isobutoxysulfonyl, tert.-Butoxysulfonyl, Hexoxysulfonyl, Dodecyloxysulfonyl, Octadecyloxysulfonyl, Phenoxysulfonyl, 1- und 2-Naphthyloxysulfonyl, (4-tert.-Butylphenoxy)-sulfonyl und (4-Chlorphenoxy)sulfonyl;
Diphenylphosphino, Di-(o-tolyl)phosphino und Diphenylphosphinoxido;
Halogene sind Chlor, Brom und Iod;
Aryl- bzw. Hetarylazo sind beispielsweise Phenylazo, 2-Napthylazo, 2-Pyridylazo und 2-Pyrimidylazo;
Gegebenenfalls substituierte Cycloalkyle sind beispielsweise Cyclopropyl, Cyclobutyl, Cyclopentyl, 2- und 3-Methylcyclopentyl, 2- und 3-Ethylcyclo-pentyl, Cyclohexyl, 2-, 3- und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methyl-cycloheptyl, 2-, 3- und 4-Ethylcycloheptyl, 3- und 4-Propylcycloheptyl, 3- und 4-Iso-propylcycloheptyl, 3- und 4-Butylcycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4- und 5-Ethylcyclooctyl und 3-, 4- und 5-Propylcyclooctyl; 3- und 4-Hydroxycyclohexyl, 3- und 4- Nitrocyclohexyl und 3- und 4-Chlorcyclohexyl. Die als Indizes angegebenen Zahlen nach dem Symbol "C" beziehen sich auf die Mindest- und Höchstanzahl der C-Atome der Cycloalkyle.
Beispiele für gegebenenfalls unterbrochene Cycloalkyle sind
1-, 2- und 3-Cyclopentenyl, 1-, 2-, 3- und 4-Cyclohexenyl, 1-, 2- und 3-Cycloheptenyl und 1-, 2-, 3- und 4-Cyclooctenyl;
2-Dioxanyl, 1-Morpholinyl, 1-Thiomorpholinyl, 2- und 3-Tetrahydrofuryl, 1-, 2- und 3-Pyrrolidinyl, 1-Piperazyl, 1-Diketopiperazyl und 1-, 2-, 3- und 4-Piperidyl;
Gegebenenfalls kondensierte und/oder substituierte und/oder unterbrochene Aryl- und Hetarylgruppen sollten mindestens 3 bis 14 Ringatome aufweisen, vorzugsweise 5 bis 10 Ringatome, und sind beispielsweise
Phenyl, 2-Naphthyl, 2- und 3-Pyrryl, 2-, 3- und 4-Pyridyl, 2-, 4- und 5-Pyrimidyl, 3-, 4-und 5-Pyrazolyl, 2-, 4- und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothia-zolyl, 5-Benzothiadiazolyl, 2- und 5-Benzimidazolyl und 1- und 5-Isochinolyl;
1-, 2-, 3-, 4-, 5-, 6- und 7-Indolyl, 1-, 2-, 3-, 4-, 5-, 6- und 7-Isoindolyl, 5-(4-Methyliso-indolyl), 5-(4-Phenylisoindolyl), 1-, 2-, 4-, 6-, 7- und 8-(1,2,3,4-Tetrahydroisochinolinyl), 3-(5-Phenyl)-(1,2,3,4-tetrahydroisochinolinyl), 5-(3-Dodecyl-(1,2,3,4-tetrahydroisochinolinyl), 1-, 2-, 3-, 4-, 5-, 6-, 7- und 8-(1,2,3,4-Tetrahydrochinolinyl) und 2-, 3-, 4-, 5-, 6-, 7- und 8-Chromanyl, 2-, 4- und 7-Chinolinyl, 2-(4-Phenylchinolinyl) und 2-(5-Ethylchinolinyl);
2-, 3- und 4-Methylphenyl, 2,4-, 3,5- und 2,6-Dimethylphenyl, 2,4,6-Trimethylphenyl, 2-, 3- und 4-Ethylphenyl, 2,4-, 3,5- und 2,6-Diethylphenyl, 2,4,6-Triethylphenyl, 2-, 3- und 4-Propylphenyl, 2,4-, 3,5- und 2,6-Dipropylphenyl, 2,4,6-Tripropylphenyl, 2-, 3- und 4-Isopropylphenyl, 2,4-, 3,5- und 2,6-Diisopropylphenyl, 2,4,6-Trüsopropylphenyl, 2-, 3-und 4-Butylphenyl, 2,4-, 3,5- und 2,6-Dibutylphenyl, 2,4,6-Tributylphenyl, 2-, 3- und 4-Isobutylphenyl, 2,4-, 3,5- und 2,6-Diisobutylphenyl, 2,4,6-Triisobutylphenyl, 2-, 3- und 4-sec.-Butylphenyl, 2,4-, 3,5- und 2,6-Di-sec.-butylphenyl und 2,4,6-Tri-sec.-butyl-phenyl; 2-, 3- und 4-Methoxyphenyl, 2,4-, 3,5- und 2,6-Dimethoxyphenyl, 2,4,6-Tri-methoxyphenyl, 2-, 3- und 4-Ethoxyphenyl, 2,4-, 3,5- und 2,6-Diethoxyphenyl, 2,4,6-Triethoxyphenyl, 2-, 3- und 4-Propoxyphenyl, 2,4-, 3,5- und 2,6-Dipropoxyphenyl, 2-, 3- und 4-Isopropoxyphenyl, 2,4- und 2,6-Diisopropoxyphenyl und 2-, 3- und 4-Butoxy-phenyl; 2-, 3- und 4-Chlorphenyl und 2,4-, 3,5- und 2,6-Dichlorphenyl; 2-, 3- und 4-Hydroxyphenyl und 2,4-, 3,5- und 2,6-Dihydroxyphenyl; 2-, 3- und 4-Cyanophenyl; 3- und 4-Carboxyphenyl; 3- und 4-Carboxamidophenyl, 3- und 4-N-Methylcarboxamido-phenyl und 3- und 4-N-Ethylcarboxamidophenyl; 3- und 4-Acetylaminophenyl, 3- und 4-Propionylaminophenyl und 3- und 4-Buturylaminophenyl; 3- und 4-N-Phenylamino-phenyl, 3- und 4-N-(o-Tolyl)aminophenyl, 3- und 4-N-(m-Tolyl)aminophenyl und 3- und 4-(p-Tolyl)aminophenyl; 3- und 4-(2-Pyridyl)aminophenyl, 3- und 4-(3-Pyridyl)amino-phenyl, 3- und 4-(4-Pyridyl)aminophenyl, 3- und 4-(2-Pyrimidyl)aminophenyl und 4-(4-Pyrimidyl)aminophenyl;
4-Phenylazophenyl, 4-(1-Naphthylazo)phenyl, 4-(2-Naphthylazo)phenyl, 4-(4-Naphthylazo)phenyl,4-(2-Pyriylazo)phenyl, 4-(3-Pyridylazo)phenyl, 4-(4-Pyridylazo)phenyl, 4-(2-Pyrimidylazo)phenyl, 4-(4-Pyrimidylazo)phenyl und 4-(5-Pyrimidylazo)phenyl;
Phenoxy, Phenylthio, 2-Naphthoxy, 2-Naphthylthio, 2-, 3- und 4-Pyridyloxy, 2-, 3- und 4-Pyridylthio, 2-, 4- und 5-Pyrimidyloxy und 2-, 4- und 5-Pyrimidylthio.

Vorzugsweise handelt es sich bei den erfindungsgemäßen Diimiden der allgemeinen Formeln (I) und (la) oder Mischungen davon um solche, bei denen alle R^{A} die gleiche Bedeutung besitzen.

Ebenfalls bevorzugt handelt es sich bei den erfindungsgemäßen Diimiden der allgemeinen Formeln (I) und (la) oder Mischungen davon um solche, bei denen jedes R^{A} unabhängig voneinander Aryloxy oder Arylthio ist, wobei das gesamte Ringsystem ein- oder mehrfach durch die Reste (i), (ii), (iii), (iv) und/oder (v) wie oben angegeben, substituiert sein kann. Insbesondere bevorzugt ist, wenn R^{A} unabhängig voneinander ein- oder mehrfach durch einen Rest (i) substituiert sein kann.

Ebenfalls bevorzugt handelt es sich bei den erfindungsgemäßen Dümiden der allgemeinen Formeln (I) und (Ia) oder Mischungen davon um solche, bei denen jedes R^{A} unabhängig voneinander 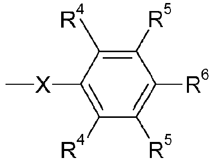 ist,
wobei
X O oder S ist und
R⁴, R⁵, R⁶ unabhängig voneinander Wasserstoff oder die Reste (i), (ii), (iii), (iv) und/oder (v) wie oben angegeben mit der Maßgabe sein können, dass zumindest einer der Reste R⁴, R⁶ kein Wasserstoff ist. Insbesondere bevorzugt ist, dass wenn R⁴ C₁-C₃₀ Alkyl oder C₃-C₈ Cycloalkyl ist, in der 1-Position kein ternäres Kohlenstoffatom auftritt.

Weiterhin bevorzugt ist, dass beide R⁴ kein Wasserstoff und R⁵, R⁶ Wasserstoff sind oder dass R⁶ kein Wasserstoff ist und R⁴, R⁵ Wasserstoff sind.

Ebenfalls bevorzugt handelt es sich bei den erfindungsgemäßen Diimiden der allgemeinen Formeln (I) und (la) oder Mischungen davon um solche, bei denen jedes R' unabhängig voneinander C₁-C₃₀ Alkyl oder Aryl ist, wobei das gesamte Ringsystem ein- oder mehrfach durch die Reste (i), (ii), (iii), (iv) und/oder (v) wie oben angegeben, substituiert sein kann. Insbesondere bevorzugt ist R' ein- oder mehrfach durch einen Rest (i) substituiert. Ebenfalls bevorzugt sind alle R' gleich.

Die erfindungsgemäßen Diimide der allgemeinen Formel (I) und (la) zeigen starke Absorption im Infrarotbereich bei Wellenlängen von 700 bis 1100 nm.

Sie eignen sich daher ebenso wie deren Vorstufen (Ib) und (Ic) für eine Vielzahl von Anwendungen, wie die Einfärbung von hochmolekularen organischen und anorganischen Materialien, z.B. von Lacken, Druckfarben und Kunststoffen, zur Herstellung im nahinfraroten Bereich des elektromagnetischen Spek-trums absorbierender wäßriger Polymerisatdispersionen, zur Erzeugung für das menschliche Auge nicht sichtbarer, Infrarotlicht absorbierender Markierungen und Beschriftungen, als Infrarotabsorber für das Wärmemanagement, als IR-laserstrahlen-absorbierende Materialien beim Schweißbehandeln von Kunststoffteilen, als Halbleiter in der organischen Elektronik, als Emitter in Elektro- und Chemilumineszenzanwendungen sowie als Aktivkomponenten in der Photovoltaik.

Ein weiterer Gegenstand der vorliegenden Erfindung liegt daher in der Verwendung eines erfindungsgemäßen Pentarylentetracarbonsäurediimids bzw. deren Vorstufe oder Mischungen davon zur Einfärbung von hochmolekularen organischen und anorganischen Materialien, als Dispergierhilfsmittel und Pigmentadditive für organische Pigmente, zur Herstellung im nahinfraroten Bereich des elektromagnetischen Spetrums absorbierender wässriger Polymerisatdispersionen, zur Erzeugung für das menschliche Auge nicht sichtbarer, Infrarotlicht absorbierender Markierungen und Beschriftungen, als Infrarotabsorber für das Wärmemanagement, als IR-laserstrahlabsorbierende Materialien beim Schweißbehandeln von Kunststoffteilen, als Halbleiter in der organischen Elektronik, als Emitter in Elektro- und Chemilumineszenzanwendungen oder als Aktivkomponente in der Photovoltaik.

### Beispiele

### Beispiel 1

### Herstellung von N-(2,6-Diisopropylphenyl-1,6,9,14-tetra[2,6-diisopropyl phenoxy] -11-(4,4,5,5-tetramethyl-1,3,2-dioxaboran-2-yl)- terrylen-3,4-dicarbonsäureimid

Zu einer Lösung von 0,72 g (0,52 mmol) N-(2,6-Diisopropylphenyl-1,6,9,14-tetra[2,6-diisopropyl phenoxy] -11-bromo- terrylen-3,4-dicarbonsäureimid in 25 ml wasserfreiem Toluol in einem 50 ml-Schlenckrohr werden nacheinander 0,33 g (1,3 mmol) Bis(pinacolato)diboran, 0,2 g (2,0 mmol) Natriumacetat und 0,2 g (0,26 mmol) [1,1'-Bis(diphenylphosphino)ferrocen]palladium(II)chlorid zugegeben. Die erhaltene Mischung wird dann unter Argon auf 70°C erhitzt und über Nacht bei dieser Temperatur gehalten. Nach Abkühlen auf Raumtemperatur wird das Produkt mit Methylenchlorid extrahiert und mit Wasser gewaschen. Dann wird das Lösungsmittel abdestilliert. Der feste Rückstand wird einer Säulenfiltration an Kieselgel mit Ethylacetat/Hexans 1:20 als Eluens unterzogen.

Es werden 0,63 g Produkt in Form eines blauen Feststoffs erhalten, was einer Ausbeute von 84% entspricht.

### Analytische Daten:

¹H-NMR (500 MHz, CD₂Cl₂, 25°C): δ = 9,79 (m, 2H), 9,60 (m, 2H), 8,62 (d, 1H), 7,71 (s, 2H), 7,39 (T, 1H), 7,40-7,30 (m, 13H), 7,28 (d, 2H), 6,28 (d, 1H), 3,15(m, 8H), 2.68 (m, 2H), 1,36 (s, 12H), 1,26 (d, 24 H), 1,09 (d, 24 H), 1,0 (d, 12H,)ppm;
UV-Vis (CHCl₃): λₘₐₓ = 672 nm;
MS (Maldi): m/z (rel. Int.) = 1435,7 (100%) [M+].

### Beispiel 2

### Herstellung von N-(2,6-Diisopropylphenyl-1,6,9,14-tetra[2,6-diisopropylphenoxy]-11-(9-[N-(2,6-diisopropylphenyl)]-perylen-3,4-dicarbonsäureimid)terrylen-3,4-dicarbonsäureimid

Zu einer unter N₂ gerührten Mischung von 0,143 g (0,1 mmol) N-(2,6-Diisopropylphenyl-1,6,9,14-tetra[2,6-diisopropyl-phenoxy]-11-(4,4,5,5-tetramethyl-1,3,2-dioxaboran-2-yl)-terrylen-3,4-dicarbonsäureimid und 0,084 g (0,15 mmol) N-(2,6-Diisopropylphenyl-9-bromo-perylen-3,4-dicarbonsäureimid in 15 ml Toluol werden zuerst eine Lösung von 0,038 g (0,28 mmol) Kaliumcarbonat in 4 ml Wasser und 0,4 mL Ethanol und dann 0,001 g (0,004 mmol) Palladium(II)-acetat und 0,008 g (0,003 mmol) 2-Dicyclohexylphosphino-2,6-dimethoxybiphenyl zugegeben. Die Mischung wird unter N₂ auf 90°C erhitzt und 14 h bei dieser Temperatur gerührt. Nach Abkühlen auf Raumtemperatur wird die organische Phase abgetrennt und das Lösungsmittel im Vakuum abgezogen. Das Rohprodukt wird einer Säulenchromatographie an Kieselgel mit Toluol als Eluens unterzogen.

Es werden 116 mg Produkt in Form eines schwarzgrünen Feststoffs erhalten, was einer Ausbeute von 65% entspricht.

### Analytische Daten:

MS (Maldi): m/z (rel. Int.) = 1790,2(100%) [M+].

### Beispiel 3

### Cyclodehydrierung zum N,N'-Bis(2,6-diisopropylphenyl)-1,6,9,22-tetra(2,6-diisopropylphenyl phenoxy)pentarylen-3,4:15,16-tetracarbonsäuredimid

Eine Mischung von 0,05 g (0,028 mmol) des Produktes aus Beispiel 2, 0,10 g (0,72 mmol) Kaliumcarbonat, 2,0 mL Ethanolamin und 1,0 mL Diethylglykoldiethylether wird auf 120°C unter Stickstoffatmosphäre erhitzt. Nach 48 h ist das Edukt komplett umgesetzt.

Nach Abkühlen wird das Reaktionsprodukt aus Wasser gefällt, abfiltriert, mit heißem Wasser und abschließend mit Hexan gewaschen, bis der Ablauf farblos wird. Der Rückstand wird über Nacht einer Soxleth-Extraktion mit Hexan unterzogen. Das Produkt wird im Vakuum bei 70°C getrocknet.

Es werden 30 mg Produkt in Form eines schwarzgrünen Feststoffs erhalten, was einer Ausbeute von 61% entspricht.

### Analytische Daten:

UV-Vis (CH₂Cl₂): λₘₐₓ = 871, 780 nm;
MS (Maldi): m/z (rel. Int.) = 1788,1 (100%) [M+].

## Patentansprüche

1. Pentarylentetracarbonsäurediimid der Formel (I) oder (la) oder Mischungen da von 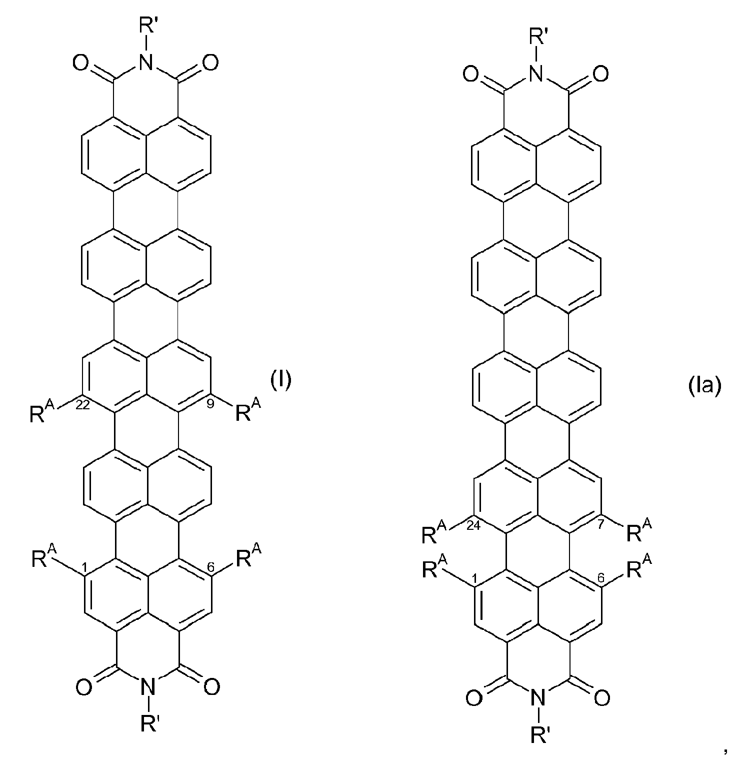 wobei
jedes R^{A} unabhängig voneinander gleich oder verschieden der folgende Rest ist:
H;
Aryloxy, Arylthio, Hetaryloxy oder Hetarylthio, an das jeweils weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CO-, -SO- und/oder -SO₂- unterbrochen sein kann, anneliert sein können, wobei das gesamte Ringsystem ein- oder mehrfach durch die Reste (i), (ii), (iii), (iv) und/oder (v) substituiert sein kann:
(i) C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂ unterbrochen sein kann und das ein- oder mehrfach substituiert sein kann durch: C₁-C₁₂-Alkoxy, C₁-C₆-Alkylthio, -C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³ ,-POR²R³, Aryl und/oder gesättigtes oder ungesättigtes C₄-C₇-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, wobei die Aryl- und Cycloalkylreste jeweils ein- oder mehrfach durch C₁-C₁₈-Alkyl und/oder die vorstehenden, als Substituenten für Alkyl genannten Reste substituiert sein können;
(ii) C₃-C₈-Cycloalkyl, dessen Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann und an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, - CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂Alkoxy, C₁-C₆-Alkylthio, - C≡CR¹, -CR¹=CR¹₂, Hydroxy, Mercapto, Halogen, Cyano, Nitro, -NR²R³, -NR²COR³, -CONR²R³, -SO₂NR²R³, -COOR², -SO₃R², -PR²R³ und/oder-POR²R³;
(iii) Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem ein- oder mehrfach substituiert sein kann durch: C₁-C₁₈-Alkyl, C₁-C₁₂-Alkoxy, C₁-C₆-Alkylthio, -C≡CR¹ -CR¹≡CR¹₂, Hydroxy, Mercapto, Halogen, Cyano, Nitro, -NR²R³, -N_{R}²COR³, - CONR²R³, -SO₂NR²R³, -COOR², -SO₃R², -PR²R³, -POR²R³, Aryl und/oder Hetaryl, das jeweils durch C₁-C₁₈-Alkyl, C₁-C₁₂-Alkoxy, Hydroxy, Mercapto, Halogen, Cyano, Nitro, -NR²R³, -NR²COR³, -CONR²R³, - SO₂NR²R³, -COOR², -SO₃R², -PR²R³ und/oder -POR²R³ substituiert sein kann;
(iv) ein Rest -U-Aryl, der ein- oder mehrfach durch die vorstehenden, als Substituenten für die Arylreste (iii) genannten Reste substituiert sein kann, wobei U eine Gruppierung -O-, -S-, -NR¹-, -CO-, -SO- oder -SO₂-bedeutet;
(v) C₁-C₁₂Alkoxy, C₁-C₆-Alkylthio, -C≡CR¹ -CR¹=CR¹₂, Hydroxy, Mercapto, Halogen, Cyano, Nitro, -NR²R³, -NR²COR³, -CONR²R³, - SO₂NR²R³, -COOR², -SO₃R², -PR²R³ und/oder-POR²R³;
R¹ Wasserstoff oder C₁-C₁₈-Alkyl, wobei die Reste R¹ gleich oder verschieden sein können, wenn sie mehrfach auftreten, ist;
R², R³ unabhängig voneinander Wasserstoff; C₁-C₁₈-Alkyl, dessen Kohlenstoffkette durch eine oder mehrere Gruppierungen -O-, -S-, -CO-, -SO- und/oder-SO₂- unterbrochen sein kann und das ein- oder mehrfach durch C₁-C₁₂-Alkoxy, C₁-C₆-Alkylthio, Hydroxy, Mercapto, Halogen, Cyano, Nitro und/oder -COOR¹ substituiert sein kann;
Aryl oder Hetaryl, an das jeweils weitere gesättigte oder ungesättigte 5-bis 7-gliedrige Ringe, deren Kohlenstoffgerüst durch eine oder mehrere Gruppierungen -O-, -S-, -CO- und/oder -SO₂- unterbrochen sein kann, anneliert sein können, wobei das gesamte Ringsystem ein- oder mehrfach durch C₁-C₁₂Alkyl und/oder die vorstehenden, als Substituenten für Alkyl genannten Reste substituiert sein kann, sind;
jedes R' unabhängig voneinander Wasserstoff;
C₁-C₃₀-Alkyl, dessen Kohlenstoffkette durch eine oder mehrere Gruppierungen -O-, -S-, -NR¹-, -N=CR¹-, -C≡C-, -CR¹=CR¹-, -CO-, -SO- und/oder -SO₂- unterbrochen sein kann und das ein- oder mehrfach durch die als Substituenten für die Reste R^{A} genannten Reste (ii), (iii), (iv) und/oder (v) substituiert sein kann;
C₃-0₈-Cycloalkyl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem durch die als Substituenten für die Reste R^{A} genannten Reste (i), (ii), (iii), (iv) und/oder (v) substituiert sein kann; oder Aryl oder Hetaryl, an das weitere gesättigte oder ungesättigte 5- bis 7-gliedrige Ringe, deren Kohlenstoffgerüst durch eine oder mehrere Gruppierungen -O-, -S-, -NR¹-, -N=CR¹-, -CR¹=CR¹-, -CO-, -SO- und/oder -SO₂- unterbrochen sein kann, anneliert sein können, wobei das gesamte Ringsystem substituiert sein kann durch die als Substituenten für die Reste R^{A} genannten Reste (i), (ii), (iii), (iv), (v), Aryl- und/oder Hetarylazo, das jeweils durch C₁-C₁₀-Alkyl, C₁-C₆-Alkoxy und/oder Cyano substituiert sein kann, ist;
mit der Maßgabe, dass mindestens drei R^{A} in Formel (I) bzw. (la) verschieden von H sind.

2. Pentarylentetracarbonsäurediimid nach Anspruch 1 oder Mischungen davon, wobei alle vier R^{A} in Formel (I) bzw. (la) verschieden von H sind.

3. Pentarylentetracarbonsäurediimid nach Anspruch 1 oder Mischungen davon, wobei R^{A} in 22-Position in Formel (I) oder R^{A} in 24-Position in Formel (la) H ist.

4. Pentarylentetracarbonsäurediimid nach einem der Ansprüche 1 bis 3 oder Mischungen davon gemäß Formel (I).

5. Verfahren zur Herstellung von Pentarylentetracarbonsäurediimiden nach einem der Ansprüche 1 bis 4, die Schritte enthaltend
(a) Kupplung mindestens einer Terrylenverbindung der Formel (II) bzw. (IIa) 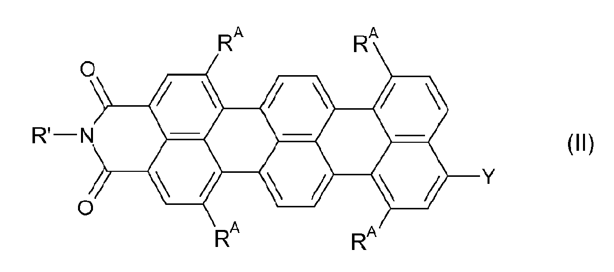 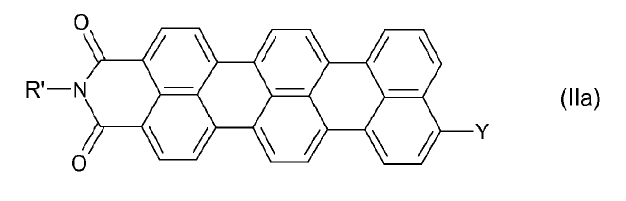 mit mindestens einer Verbindung der Formel (III) bzw. (IIIa) 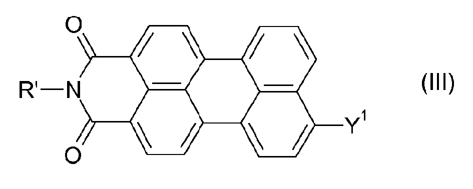 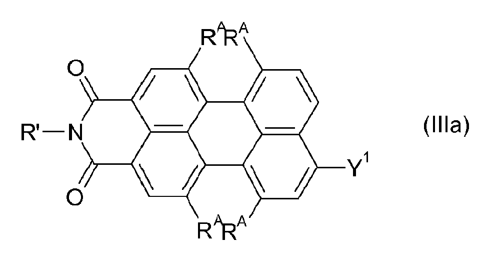 wobei
Y, Y¹ beide Halogen sind oder ein Rest von Y, Y¹ Halogen und der andere B(OR")₂ ist;
jedes R" unabhängig voneinander Wasserstoff, C₁-C₃₀-Alkyl, C₅-C₈-Cycloalkyl, Aryl oder Hetaryl oder miteinander verbunden unter Ausbildung eines die beiden Sauerstoffatome sowie das Boratom enthaltenden 5- bis 7-gliedrigen Rings, an den ungesättigte oder gesättigte Ringe anneliert sein können und der an den Kohlenstoffatomen durch bis zu 4 C₁-C₃₀-Alkyl-, C₅-C₈-Cycloalkyl-, Aryl- oder Hetarylgruppen substituiert sein kann, ist;
jedes R' und R^{A} die Bedeutung wie in Anspruch 1 besitzt;
(b) Cyclodehydrierung des in Schritt (a) erhaltenen Umsetzungsproduktes zu einer Pentarylenverbindung der allgemeinen Formel (I) oder (la) oder Mischungen davon.

6. Pentarylentetracarbonsäurediimidvorstufe der Formel (Ib) oder (Ic) oder Mischungen davon 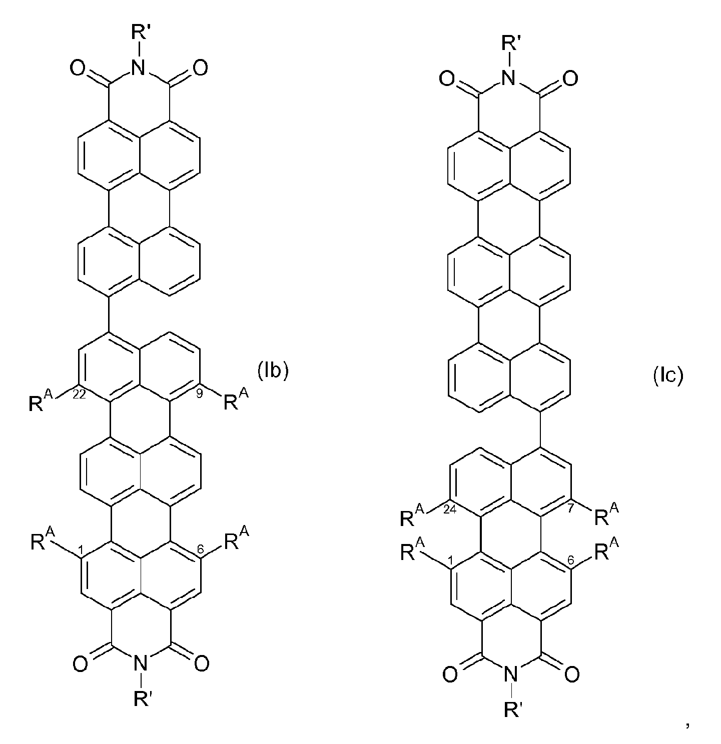 wobei jedes R' und R^{A} die Bedeutung wie in Anspruch 1 besitzt.

7. Verwendung eines Pentarylentetracarbonsäurediimids bzw. Vorstufen oder Mischungen davon nach einem der Ansprüche 1 bis 4 und 6 zur Einfärbung von hochmolekularen organischen und anorganischen Materialien, als Dispergierhilfsmittel und Pigmentadditive für organische Pigmente, zur Herstellung im nahinfraroten Bereich des elektromagnetischen Spektrums absorbierender wässriger Polymerisatdispersionen, zur Erzeugung für das menschliche Auge nicht sichtbarer, Infrarotlicht absorbierender Markierungen und Beschriftungen, als Infrarotabsorber für das Wärmemanagement, als IR-laserstrahlenabsorbierende Materialien beim Schweißbehandeln von Kunststoffteilen, als Halbleiter in der organischen Elektronik, als Emitter in Elektro- und Chemilumineszenzanwendungen oder als Aktivkomponente in der Photovoltaik.

## Claims

1. A pentarylenetetracarboximide of the formula (I) or (Ia) or mixtures thereof 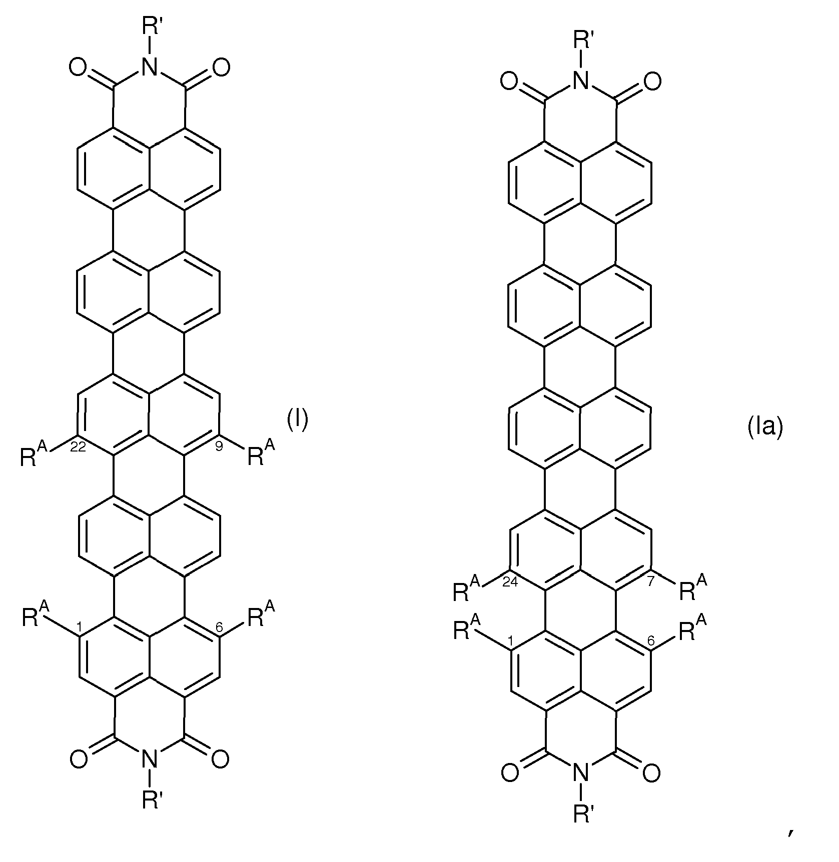 where
each R^{A} is the same or different and is independently the following radical:
H;
aryloxy, arylthio, hetaryloxy or hetarylthio, to each of which may be fused further saturated or unsaturated 5- to 7-membered rings whose carbon skeleton may be interrupted by one or more -O-, -S-, -NR¹-, -N=CR¹-, -CO-, -SO- and/or -SO₂- moieties, where the entire ring system may be mono- or polysubstituted by the (i), (ii), (iii), (iv) and/or (v) radicals:
(i) C₁-C₃₀-alkyl whose carbon chain may be interrupted by one or more -O-, -S-, -NR¹-, -N=CR¹-, -C≡C-, -CR¹=CR¹-, -CO-, -SO- and/or -SO₂- moieties and which may be mono- or polysubstituted by: C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR², -SO₃R², -PR²R³, -POR²R³, aryl and/or saturated or unsaturated C₄-C₇-cycloalkyl whose carbon skeleton may be interrupted by one or more -0-, -S-, -NR¹-, -N=CR¹-, -CR¹=CR¹-, -CO-, -SO- and/or -SO₂- moieties, where the aryl and cycloalkyl radicals may each be mono- or polysubstituted by C₁-C₁₈-alkyl and/or the above radicals specified as substituents for alkyl;
(ii) C₃-C₈-cycloalkyl whose carbon skeleton may be interrupted by one or more -O-, -S-, -NR¹-, -N=CR¹-, -CR¹=CR¹-, -CO-, -SO- and/or -SO₂- moieties and to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂-moieties, where the entire ring system may be mono- or polysubstituted by: C₁-C₁₈-alkyl, C₁-C₁₂-alkoxy, C₁-C₆-alkylthio, -C≡CR¹, -CR¹=CR¹_{2,} hydroxyl, mercapto, halogen, cyano, nitro, -NR²R³, -NR²COR³, -CONR²R³, -SO₂NR²R³, -COOR² -SO₃R² -PR²R³ and/or -POR²R³.
(iii) aryl or hetaryl to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂-moieties, where the entire ring system may be mono- or polysubstituted by: C₁-C₁₈-alkyl, C₁-C₁₂-alkoxy, C₁-C₆-alkylthio, -C≡CR¹, -CR¹=CR¹₂, hydroxyl, mercapto, halogen, cyano, nitro, -NR²R³, -NR²COR³, -CONR²R³, -SO₂NR²R³, -COOR², -SO₃R², -PR²R³, -POR²R³, aryl and/or hetaryl, each of which may be substituted by C₁-C₁₈-alkyl, C₁-C₁₂-alkoxy, hydroxyl, mercapto, halogen, cyano, nitro, -NR²R³, -NR²COR³, -CONR²R³, -SO₂NR²R³, -COOR², -SO₃R², -PR²R³ and/or -POR²R³ ;
(iv) a -U-aryl radical which may be mono- or polysubstituted by the above radicals specified as substituents for the aryl radicals (iii), where U is a -O-, -S-, -NR¹-, -CO-, -SO- or -SO₂- moiety;
(v) C₁-C₁₂-alkoxy, C₁-C₆-alkylthio, -C≡CR¹, -CR¹≡CR¹₂, hydroxyl, mercapto, halogen, cyano, nitro, -NR²R³, -NR²COR³, -CONR²R³, -SO₂NR²R³, -COOR² -SO₃R², -PR²R³ and/or -POR²R³.
R¹ is hydrogen or C₁-C₁₈-alkyl, where the R¹ radicals may be the same or different when they occur more than once;
R² R³ are each independently hydrogen; C₁-C₁₈-alkyl whose carbon chain may be interrupted by one or more -O-, -S-, -CO-, -SO- and/or -SO₂- moieties and which may be mono- or polysubstituted by C₁-C₁₂-alkoxy, C₁-C₆-alkylthio, hydroxyl, mercapto, halogen, cyano, nitro and/or -COOR¹;
aryl or hetaryl, to each of which may be fused further saturated or unsaturated 5- to 7-membered rings whose carbon skeleton may be interrupted by one or more -O-, -S-, -CO-and/or -SO₂- moieties, where the entire ring system may be mono- or polysubstituted by C₁-C₁₂-alkyl and/or the above radicals specified as substituents for alkyl;
each R' is independently hydrogen;
C₁-C₃₀-alkyl whose carbon chain may be interrupted by one or more -O-, -S-, -NR¹-, -N=CR¹-, -C≡C-, -CR¹≡CR¹-, -CO-, -SO- and/or -SO₂- moieties and which may be mono- or polysubstituted by the (ii), (iii), (iv) and/or (v) radicals specified as substituents for the R^{A} radicals;
C₃-C₈-cycloalkyl to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂- moieties, where the entire ring system may be substituted by the (i), (ii), (iii), (iv) and/or (v) radicals specified as substituents for the R^{A} radicals; or
aryl or hetaryl to which may be fused further saturated or unsaturated 5- to 7-membered rings whose carbon skeleton may be interrupted by one or more -O-, -S-, -NR¹-, -N=CR¹-, -CR¹=CR¹-, -CO-, -SO- and/or -SO₂-moieties, where the entire ring system may be substituted by the (i), (ii), (iii), (iv), (v) radicals specified as substituents for the R^{A} radicals, aryl- and/or hetarylazo, each of which may be substituted by C₁-C₁₀-alkyl, C₁-C₆-alkoxy and/or cyano;
with the proviso that at least three R^{A} in formula (I) or (Ia) are different than H.

2. A pentarylenetetracarboximide according to claim 1 or mixtures thereof, where all four R^{A} in formula (I) or (Ia) are different than H.

3. A pentarylenetetracarboximide according to claim 1 or mixtures thereof, where R^{A} in the 22 position in formula (I) or R^{A} in the 24 position in formula (Ia) is H.

4. A pentarylenetetracarboximide according to any one of claims 1 to 3 or mixtures thereof of the formula (I).

5. A process for preparing pentarylenetetracarboximides according to any one of claims 1 to 4, comprising the steps of
(a) coupling at least one terrylene compound of the formula (II) or (IIa) 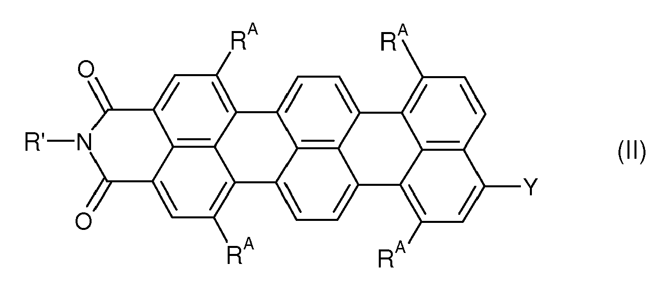 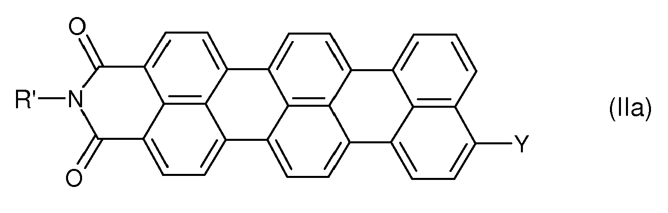 with at least one compound of the formula (III) or (IIIa) 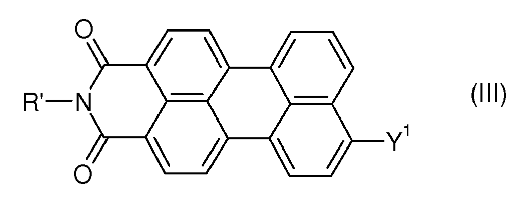 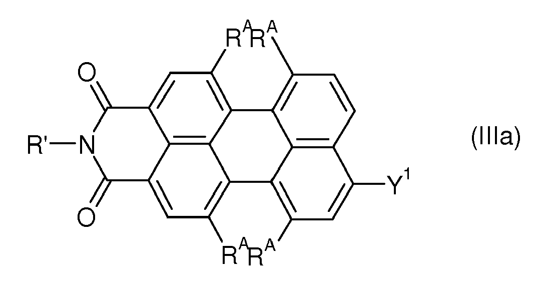 where
Y, Y¹ are each halogen or one radical of Y, Y¹ is halogen and the other is B(OR")₂;
each R" is independently hydrogen, C₁-C₁₀-alkyl, C₅-C₅-cycloalkyl, aryl or hetaryl or, joined together to form a 5- to 7-membered ring comprising the two oxygen atoms and the boron atom, may be fused to the unsaturated or saturated rings, where said ring may be substituted on the carbon atoms by up to 4 C₁-C₃₀-alkyl, C₅-C₅-cycloalkyl, aryl or hetaryl groups;
each R' and R^{A} is as defined in claim 1;
(b) cyclodehydrogenating the reaction product obtained in step (a) to give a pentarylene compound of the general formula (I) or (Ia) or mixtures thereof.

6. A pentarylenetetracarboximide precursor of the formula (Ib) or (Ic) or mixtures thereof 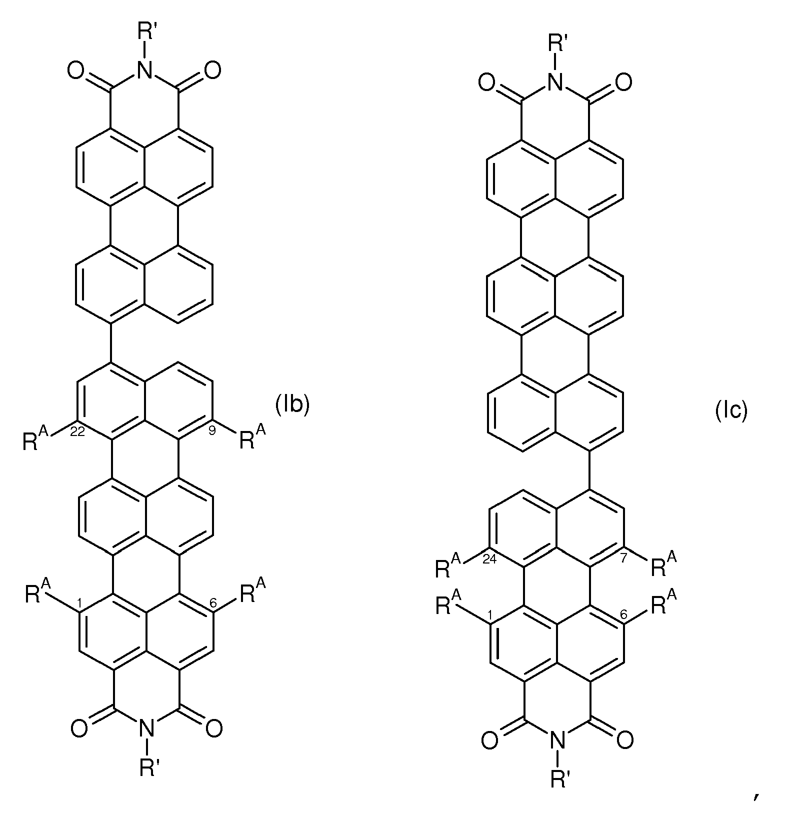 where each R' and R^{A} is as defined in claim 1.

7. The use of a pentarylenetetracarboximide or precursors or mixtures thereof according to any one of claims 1 to 4 and 6 for coloring high molecular weight organic and inorganic materials, as dispersing aids and pigment additives for organic pigments, for producing aqueous polymer dispersions which absorb in the near infrared region of the electromagnetic spectrum, for obtaining markings and inscriptions which absorb infrared light and are invisible to the human eye, as infrared absorbers for heat management, as IR laser beam absorbing materials in the fusion treatment of plastics parts, as semiconductors in organic electronics, as emitters in electro- and chemiluminescence applications or as active components in photovoltaics.

## Revendications

1. Diimide de l'acide pentarylènetétracarboxylique de formule (I) ou (Ia) ou leurs mélanges 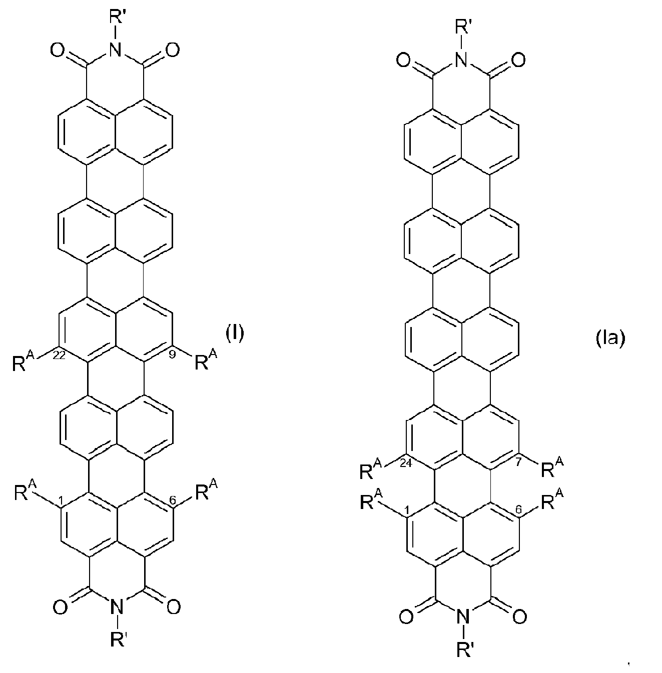 où
chaque R^{A} représente, indépendamment les uns des autres, de manière identique ou différente, le radical suivant :
H ;
aryloxy, arylthio, hétaryloxy ou hétarylthio, sur lequel à chaque fois d'autres cycles saturés ou insaturés de 5 à 7 chaînons, dont la structure carbonée peut être interrompue par un ou plusieurs groupements -O-, -S-, -NR¹-, -N=CR¹-, -CO-, -SO- et/ou -SO₂-, peuvent être condensés, l'ensemble du système cyclique pouvant être monosubstitué ou polysubstitué par les radicaux (i), (ii), (iii), (iv) et/ou (v) :
(i) C₁-C₃₀-alkyle, dont la chaîne carbonée peut être interrompue par un ou plusieurs groupements -O-, -S-, -NR¹-, -N=CR¹-, -C≡C-, -CR¹=CR¹-, -CO-, -SO- et/ou -SO₂- et qui peut être monosubstituée ou polysubstituée par : C₁-C₁₂-alcoxy, C₁-C₆-alkylthio, -C≡CR¹, -CR¹=CR¹₂, hydroxy, mercapto, halogène, cyano, nitro, -NR²R³, -NR²COR³, -CONR²R³, -SO₂NR²R³, -COOR², -SO₃R², -PR²R³, -POR²R³, aryle et/ou C₄-C₇-cycloalkyle saturé ou insaturé, dont la structure carbonée peut être interrompue par un ou plusieurs groupements -O-, -S-, -NR¹-, -N=CR¹-, -CR¹=CR¹-, -CO-, -SO- et/ou -SO₂-, les radicaux aryle et cycloalkyle pouvant à chaque fois être monosubstitués ou polysubstitués par C₁-C₁₈-alkyle et/ou les radicaux ci-dessus, mentionnés comme substituants pour alkyle pouvant être substitués ;
(ii) C₃-C₈-cycloalkyle, dont la structure carbonée peut être interrompue par un ou plusieurs groupements -O-, -S-, -NR¹-, -N=CR¹-, -CR¹=CR¹-, -CO-, -SO- et/ou -SO₂- et sur lequel d'autres cycles saturés ou insaturés de 5 à 7 chaînons, dont la structure carbonée peut être interrompue par un ou plusieurs groupements -O-, -S-, -NR¹-, -N=CR¹-, - CR¹=CR¹-, -CO-, -SO- et/ou -SO₂-, peuvent être condensés, le système cyclique total pouvant être monosubstitué ou polysubstitué par : C₁-C₁₈-alkyle, C₁-C₁₂-alcoxy, C₁-C₆-alkylthio, -C≡CR¹, -CR¹=CR¹₂, hydroxy, mercapto, halogène, cyano, nitro, -NR²R³, -NR²COR³, -CONR ²R³, -SO₂NR² R³, -COOR², -SO₃R², -PR²R³ et/ou -POR² R³ ;
(iii) aryle ou hétaryle, sur lequel d'autres cycles saturés ou insaturés de 5 à 7 chaînons, dont la structure carbonée peut être interrompue par un ou plusieurs groupements -O-, -S-, -NR¹-, -N=CR¹-, -CR¹=CR¹-, -CO-, -SO- et/ou -SO₂-, peuvent être condensés, le système cyclique total pouvant être monosubstitué ou polysubstitué par : C₁-C₁₈-alkyle, C₁-C₁₂-alcoxy, C₁-C₆-alkylthio, -C≡CR¹, -CR¹=CR¹₂, hydroxy, mercapto, halogène, cyano, nitro, -NR²R³, -NR²COR³, -CONR²R³, -SO₂NR²R³, -COOR², -SO₃R², -PR²R³, - POR²R³, aryle et/ou hétaryle, qui peut à chaque fois être substitué par C₁-C₁₈-alkyle, C₁-C₁₂-alcoxy, hydroxy, mercapto, halogène, cyano, nitro, -NR²R³, -NR²COR³, -CONR²R³, -SO₂NR²R³, -COOR², -SO₃R², -PR²R³ et/ou -POR²R³ ;
(iv) un radical -U-aryle, qui peut être monosubstitué ou polysubstitué par les radicaux ci-dessus, mentionnés comme substituants pour les radicaux aryle (iii), où U signifie un groupement -O-, -S-, -NR¹-, -CO-, -SO- ou -SO₂ ;
(v) C₁-C₁₂-alcoxy, C₁-C₆-alkylthio, -C≡CR¹, -CR¹=CR¹₂, hydroxy, mercapto, halogène, cyano, nitro, -NR²R³, -NR²COR³, -CONR²R³, - SO₂NR²R³, -COOR², -SO₃R², -PR²-R³ et/ou -POR²R³ ;
R¹ représente hydrogène ou C₁-C₁₈-alkyle, où les radicaux R¹ peuvent être identiques ou différents, lorsqu'ils existent plusieurs fois ;
R² R³ indépendamment l'un de l'autre, signifient hydrogène ;
C₁-C₁₈-alkyle, dont la chaîne carbonée peut être interrompue par un ou plusieurs groupements -O-, -S-, -CO-, -SO- et/ou -SO₂- et qui peut être monosubstitué ou polysubstitué par C₁-C₁₂-alcoxy, C₁-C₆-alkylthio, hydroxy, mercapto, halogène, cyano, nitro et/ou -COOR¹ ;
aryle ou hétaryle, sur lequel à chaque fois d'autres cycles saturés ou insaturés, de 5 à 7 chaînons, dont la structure carbonée peut être substituée par un ou plusieurs groupements -O-, -S-, -CO- et/ou -SO₂-, peuvent être condensés, l'ensemble du système cyclique pouvant être monosubstitué ou polysubstitué par C₁-C₁₂-alkyle et/ou par les radicaux ci-dessus, mentionnés comme substituants pour alkyle ;
chaque R', indépendamment les uns des autres, signifie hydrogène ;
C₁-C₃₀-alkyle, dont la chaîne carbonée peut être interrompue par un ou plusieurs groupements -O-, -S-, -NR¹-, -N=CR¹-, -C≡C-, -CR¹=CR¹-, -CO-, -SO-et/ou -SO₂- et qui peut être monosubstitué ou polysubstitué par les radicaux (ii), (iii), (iv) et/ou (v) mentionnés comme substituants pour les radicaux R^{A} _{;}
C₃-C₈-cycloalkyle, sur lequel d'autres cycles saturés ou insaturés, de 5 à 7 chaînons, dont la structure carbonée peut être interrompue par un ou plusieurs groupements -O-, -S-, -NR¹-, -N=CR¹-, -CR¹=CR¹-, -CO-, -SO- et/ou -SO₂-, peuvent être condensés, l'ensemble du système cyclique pouvant être substitué par les radicaux (i), (ii), (iii), (iv) et/ou (v) mentionnés comme substituants pour les radicaux R^{A} ; ou
aryle ou hétaryle, sur lequel à chaque fois d'autres cycles saturés ou insaturés, de 5 à 7 chaînons, dont la structure carbonée peut être interrompue par un ou plusieurs groupements -O-, -S-, -NR¹-, -N=CR¹-, -CR¹=CR¹-, -CO-, -SO- et/ou -SO₂-, peuvent être condensés, l'ensemble du système cyclique pouvant être substitué par les radicaux (i), (ii), (iii), (iv), (v) mentionnés comme substituants pour les radicaux R^{A}, arylazo et/ou hétarylazo, qui peut être à chaque fois substitué ou par C₁-C₁₀-alkyle, C₁-C₆-alcoxy et/ou cyano,
à condition qu'au moins trois R^{A} dans la formule (I) ou, selon le cas (Ia) soient différents de H.

2. Diimide de l'acide pentarylènetétracarboxylique selon la revendication 1 ou ses mélanges, les quatre R^{A} dans la formule (I) ou, selon le cas (Ia) étant différents de H.

3. Diimide de l'acide pentarylènetétracarboxylique selon la revendication 1 ou ses mélanges, R^{A} en position 22 dans la formule (I) ou R^{A} en position 24 dans la formule (Ia) représentant H.

4. Diimide de l'acide pentarylènetétracarboxylique selon l'une quelconque des revendications 1 à 3 ou ses mélanges selon la formule (I).

5. Procédé pour la préparation de diimides de l'acide pentarylènetétracarboxylique selon l'une quelconque des revendications 1 à 4, comprenant les étapes
(a) couplage d'au moins un composé terrylène de formule (II) ou, selon le cas (IIa) 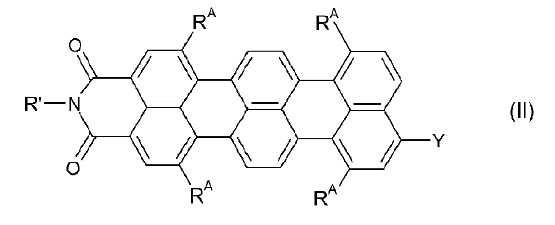 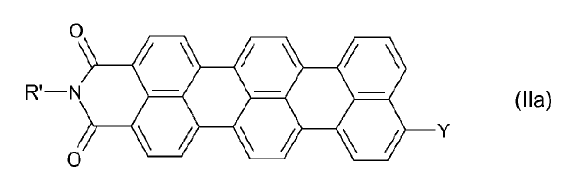 avec au moins un composé de formule (III) ou, selon le cas, (IIIa) 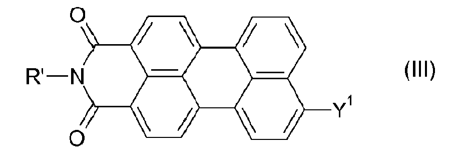 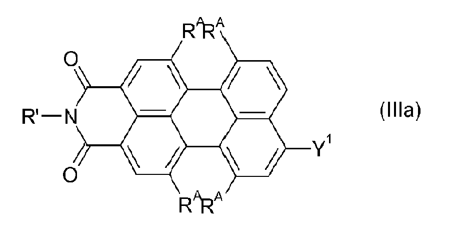 où
Y, Y¹ représentent tous deux halogène ou un radical parmi Y, Y¹ représente halogène et l'autre représente B(OR")₂ ;
chaque R" représente, indépendamment l'un de l'autre, hydrogène, C₁-C₃₀-alkyle, C₅-C₈-cycloalkyle, aryle ou hétaryle ou ils forment, en étant reliés l'un avec l'autre, un cycle de 5 à 7 chaînons, contenant les deux atomes d'oxygène ainsi que l'atome de bore, sur lequel des cycles insaturés ou saturés peuvent être condensés et qui peut être substitué, sur les atomes de carbone, par jusqu'à 4 groupes C₁-C₃₀-alkyle, C₅-C₈-cycloalkyle, aryle ou hétaryle ;
chaque R' et R^{A} présentant la signification comme dans la revendication 1 ;
(b) cyclodéshydratation du produit de transformation obtenu dans l'étape (a) en un composé pentarylène de formule générale (I) ou (Ia) ou leurs mélanges.

6. Précurseurs de diimide de l'acide pentarylènetétracarboxylique de formule (Ib) ou (Ic) ou leurs mélanges 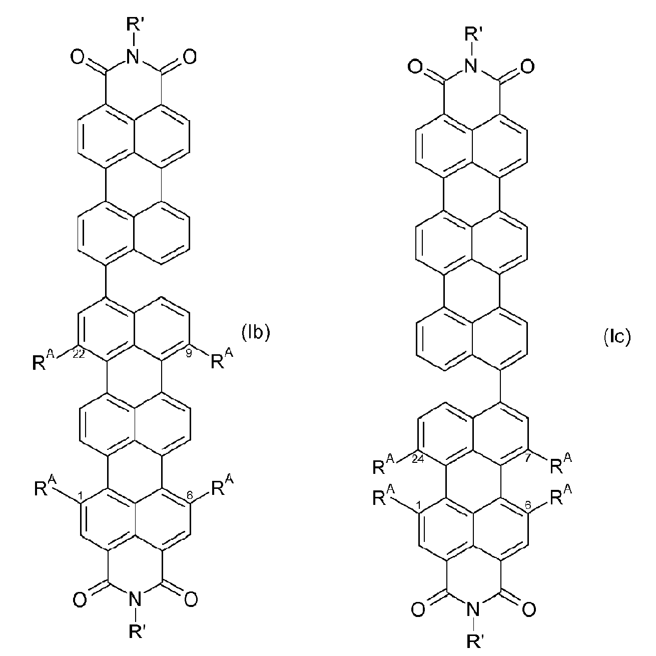 chaque R' et R^{A} présentant la signification comme dans la revendication 1.

7. Utilisation d'un diimide de l'acide pentarylènetétracarboxylique ou, selon le cas de précurseurs ou mélanges de celui-ci selon l'une quelconque des revendications 1 à 4 et 6 pour la teinture de matériaux de haut poids moléculaire, organiques et inorganiques, comme dispersants et additifs pigmentaires pour pigments organiques, pour la préparation de dispersions polymères aqueuses absorbant dans la plage des infrarouges proches du spectre électromagnétique, pour la génération de marquages et d'inscriptions invisibles pour l'oeil humain, absorbant la lumière infrarouge, comme absorbants des infrarouges pour la gestion de la chaleur, comme matériaux absorbant les rayons laser IR lors du traitement par soudure de pièces en matériau synthétique, comme semi-conducteurs dans l'électronique organique, comme émetteurs dans les utilisations électroluminescentes et chimiluminescentes ou comme composant actif dans la photovoltaïque.
